(19) Europäisches Patentamt
European Patent Office
Office européen des brevets

(11) Veröffentlichungsnummer: **0 432 600 A2**

# (12) EUROPÄISCHE PATENTANMELDUNG

(21) Anmeldenummer: **90123056.5**

(22) Anmeldetag: **01.12.90**

(51) Int. Cl.5: **C07D 213/75**, C07D 213/76, A01N 43/40, A01N 43/90, C07D 417/06, C07D 413/06, C07D 213/82

(30) Priorität: **14.12.89 DE 3941233**
**05.07.90 DE 4021439**

(43) Veröffentlichungstag der Anmeldung:
**19.06.91 Patentblatt 91/25**

(84) Benannte Vertragsstaaten:
**BE CH DE DK ES FR GB IT LI NL SE**

(71) Anmelder: **BAYER AG**

**W-5090 Leverkusen 1 Bayerwerk(DE)**

(72) Erfinder: **Andree, Roland, Dr.**
**Schillerstrasse 17**
**W-4018 Langenfeld(DE)**
Erfinder: **Fürstenwerth, Hauke, Dr.**
**Unterölbach 3a**
**W-5090 Leverkusen 3(DE)**
Erfinder: **Jelich, Klaus, Dr.**
**Paul-Ehrlich-Strasse 2**
**W-5600 Wuppertal(DE)**
Erfinder: **Beck, Gunther, Dr.**
**Am Mittelberg 19**
**W-5090 Leverkusen(DE)**
Erfinder: **Babczinski, Peter, Dr.**
**In der Lohrenbeck 11**
**W-5060 Wuppertal(DE)**
Erfinder: **Lürssen, Klaus, Dr.**
**August-Kierspel-Strasse 145**
**Bergisch-Gladbach 2(DE)**
Erfinder: **Santel, Hans-Joachim, Dr.**
**Grünstrasse 9a**
**W-5090 Leverkusen(DE)**
Erfinder: **Schmidt, Robert Rudolf, Dr.**
**Im Waldwinkel 110**
**Bergisch Gladbach 2(DE)**

## (54) 2-Iminopyridin-Derivate.

(57) Neue und bekannte 2-Iminopyridin-Derivate der allgemeinen Formel (IA)

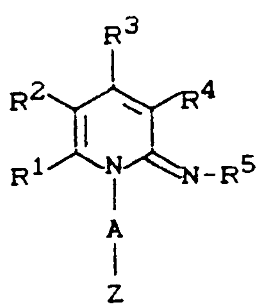

(I)

in welcher

$R^1$, $R^2$, $R^3$ und $R^4$ unabhängig voneinander für Wasserstoff, Halogen, Alkyl, Halogenalkyl, Alkoxy oder Halogenalkoxy stehen,

$R^5$ für Cyano, Alkylcarbonyl, Alkenylcarbonyl, Alkinylcarbonyl, Halogenalkylcarbonyl, Halogenalkenylcarbonyl, Halogenalkinylcarbonyl, Alkoxycarbonyl, Alkylthiocarbonyl,

EP 0 432 600 A2

für jeweils gegebenenfalls substituiertes Phenylcarbonyl oder Phenoxycarbonyl, für Alkylsulfonyl, gegebenenfalls substituiertes Phenylsulfonyl, für Alkylaminocarbonyl oder gegebenenfalls substituiertes Phenylaminocarbonyl steht,

A für Alkandiyl steht und

Z für gegebenenfalls substituiertes Aryl oder gegebenenfalls substituiertes Hetaryl steht,

und deren herbizide Verwendung sowie Verfahren zur Herstellung der neuen 2-Iminopyridin-Derivate.

## 2-IMINOPYRIDIN-DERIVATE

Die Erfindung betrifft die Verwendung von neuen und bekannten 2-Iminopyridin-Derivaten als Herbizide sowie neue 2-Iminopyridin-Derivate und mehrere Verfahren zu ihrer Herstellung.

Es ist bekannt, daß bestimmte Pyridin-Derivate wie beispielsweise das N-(2,4-Difluorphenyl)-2-[3-(trifluormethyl)-phenoxy]-3-pyridincarboxamid (bekannt aus EP-A 53 011) herbizide Wirksamkeit besitzen. Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Weiter sind bestimmte substituierte Iminopyridine bekannt (vergleiche JP 63/287764, Zit. in CA 111(1): 7429r und EP-A 259 738). Eine herbizide Wirksamkeit dieser Verbindungen ist jedoch nicht bekannt.

Zunächst werden in der vorliegenden Anmeldung die neuen 2-Iminopyridin-Derivate der Formel (I) und anschließend die neuen und bekannten 2-Iminopyridin-Derivate der Formel (IA) beschrieben.

Es wurden neue 2-Iminopyridin-Derivate der allgemeinen Formel (I),

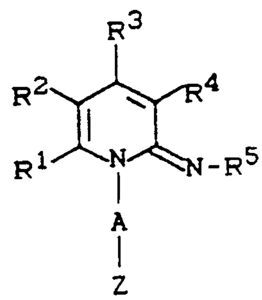

(I)

in welcher

| | |
|---|---|
| $R^1$, $R^2$, $R^3$ und $R^4$ | unabhängig voneinander für Wasserstoff, Halogen, Alkyl, Halogenalkyl, Alkoxy, Halogenalkoxy, Alkylthio oder Halogenalkylthio stehen, |
| $R^5$ | für Alkylcarbonyl, Cycloalkylcarbonyl, Alkenylcarbonyl, Alkinylcarbonyl, Halogenalkylcarbonyl, Alkoxyalkylcarbonyl, Halogenalkenylcarbonyl, Halogenalkinylcarbonyl, Alkoxycarbonyl, Alkylcarbonyloxyalkylcarbonyl, Alkoxycarbonylcarbonyl, Alkylthiocarbonyl, für jeweils gegebenenfalls substituiertes Phenylcarbonyl, Phenylalkylcarbonyl, Phenylalkenylcarbonyl, Furylcarbonyl, Thienylcarbonyl, Pyridylcarbonyl, Phenoxyalkylcarbonyl oder Phenoxycarbonyl, für Alkylsulfonyl, Halogenalkylsulfonyl, gegebenenfalls substituiertes Phenylsulfonyl, für Alkylaminocarbonyl oder gegebenenfalls substituiertes Phenylaminocarbonyl steht, |
| A | für Alkandiyl steht und |
| Z | für gegebenenfalls substituiertes Aryl oder gegebenenfalls substituiertes Hetaryl steht, |

gefunden.

Weiterhin wurde gefunden, daß man die neuen 2-Iminopyridin-Derivate der allgemeinen Formel (I),

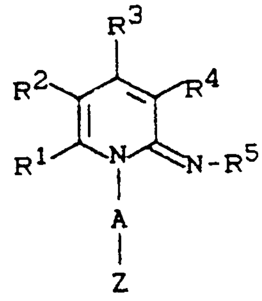

(I)

in welcher

$R^1$, $R^2$, $R^3$ und $R^4$ unabhängig voneinander für Wasserstoff, Halogen, Alkyl, Halogenalkyl, Alkoxy, Halogenalkoxy, Alkylthio oder Halogenalkylthio stehen,

$R^5$ für Alkylcarbonyl, Cycloalkylcarbonyl, Alkenylcarbonyl, Alkinylcarbonyl, Halogenalkylcarbonyl, Alkoxyalkylcarbonyl, Halogenalkenylcarbonyl, Halogen alkinylcarbonyl, Alkoxycarbonyl, Alkylcarbonyloxyalkylcarbonyl, Alkoxycarbonylcarbonyl, Alkylthiocarbonyl, für jeweils gegebenenfalls substituiertes Phenylcarbonyl, Phenylalkylcarbonyl, Phenylalkenylcarbonyl, Furylcarbonyl, Thienylcarbonyl, Pyridylcarbonyl, Phenoxyalkylcarbonyl oder Phenoxycarbonyl, für Alkylsulfonyl, Halogenalkylsulfonyl, gegebenenfalls substituiertes Phenylsulfonyl, für Alkylaminocarbonyl oder gegebenenfalls substituiertes Phenylaminocarbonyl steht,

A für Alkandiyl steht und

Z für gegebenenfalls substituiertes Aryl oder gegebenenfalls substituiertes Hetaryl steht,

erhält, wenn man 1,2-Dihydropyridiniminiumsalze der allgemeinen Formel (II),

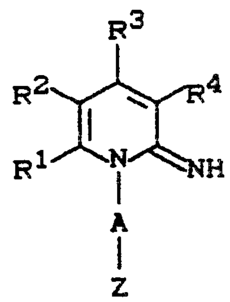 x HX (II)

in welcher

$R^1$, $R^2$, $R^3$, $R^4$, A und Z die oben angegebene Bedeutung haben und

HX für das Äquivalent einer anorganischen oder organischen Säure steht,

(a) mit Halogenverbindungen der allgemeinen Formel (III),

$$R^{5-1}-X^1 \qquad (III)$$

in welcher

$R^{5-1}$ für Alkylcarbonyl, Cycloalkylcarbonyl, Alkenylcarbonyl, Alkinylcarbonyl, Halogenalkylcarbonyl, Alkoxyalkylcarbonyl, Halogenalkenylcarbonyl, Halogenalkinylcarbonyl, Alkoxycarbonyl, Alkylthiocarbonyl, für jeweils gegebenenfalls substituiertes Phenylcarbonyl, Phenylalkylcarbonyl, Phenylalkenylcarbonyl, Furylcarbonyl, Thienylcarbonyl, Pyridylcarbonyl, Phenoxyalkylcarbonyl, Alkylcarbonyloxyalkylcarbonyl, Alkoxycarbonylcarbonyl oder Phenoxycarbonyl, für Alkylsulfonyl, Halogenalkylsulfonyl, oder gegebenenfalls substituiertes Phenylsulfonyl, steht,

$X^1$ für Halogen steht,

und für den Fall, daß $R^{5-1}$ für Alkylcarbonyl, Alkenylcarbonyl, Alkinylcarbonyl, Halogenalkylcarbonyl, Halogenalkenylcarbonyl, Halogenalkinylcarbonyl oder für gegebenenfalls substituiertes Phenylcarbonyl steht, mit den entsprechenden Carbonsäureanhydriden,

gegebenenfalls in Gegenwart einer Base und gegebenenfalls in Gegenwart eines Verdünnungsmittels umsetzt oder

(b) mit Isocyanaten der allgemeinen Formel (IV),

$$R^6-NCO \qquad (IV)$$

in welcher

$R^6$ für Alkyl oder für gegebenenfalls substituiertes Phenyl steht,

gegebenenfalls in Gegenwart einer Base und gegebenenfalls in Gegenwart eines Verdünnungsmittels

umsetzt.

Schließlich wurde gefunden, daß die neuen und bekannten 2-Iminopyridin-Derivate der allgemeinen Formel (I) bzw. (IA) sehr gute herbizide Eigenschaften aufweisen.

Die Erfindung betrifft nun die Verwendung der erfindungsgemäßen neuen 2-Iminopyridin-Derivate der Formel (1) und der bekannten 2-Iminopyridin-Derivate. Die neuen und bekannten 2-Iminopyridin-Derivate sind in der nachstehenden Formel (IA) zusammengefaßt :

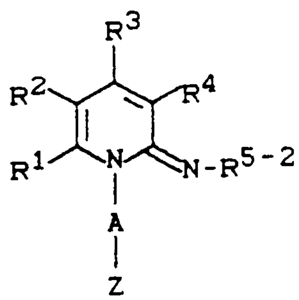

(IA)

in welcher

| | |
|---|---|
| $R^1$, $R^2$, $R^3$ und $R^4$ | unabhängig voneinander für Wasserstoff, Halogen, Alkyl, Halogenalkyl, Alkoxy, Halogenalkoxy, Alkylthio oder Halogenalkylthio stehen, |
| $R^{5-2}$ | für Cyano, Alkylcarbonyl, Cycloalkylcarbonyl, Alkenylcarbonyl, Alkinylcarbonyl, Halogenalkylcarbonyl, Alkoxyalkylcarbonyl, Halogenalkenylcarbonyl, Halogenalkinylcarbonyl, Alkoxycarbonyl, Alkylthiocarbonyl, für jeweils gegebenenfalls substituiertes Phenylcarbonyl, Phenylalkylcarbonyl, Phenylalkenylcarbonyl, Furylcarbonyl, Thienylcarbonyl, Pyridylcarbonyl, Phenoxyalkylcarbonyl, Alkylcarbonyloxyalkylcarbonyl, Alkoxycarbonylcarbonyl oder Phenoxycarbonyl, für Alkylsulfonyl, Halogenalkylsulfonyl, oder gegebenenfalls substituiertes Phenylsulfonyl, für Alkylaminocarbonyl oder gegebenenfalls substituiertes Phenylaminocarbonyl steht, |
| A | für Alkandiyl steht und |
| Z | für gegebenenfalls substituiertes Aryl oder gegebenenfalls substituiertes Hetaryl steht. |

Die Verbindungen der Formel (IA) lassen sich nach den beschriebenen Verfahren (a) und (b) und nach bekannten Methoden herstellen (vgl. z.B. EP-A 259 738).

Überraschenderweise zeigen die erfindungsgemäßen 2-Iminopyridin-Derivate der allgemeinen Formel (I) bzw. (IA) eine höhere herbizide Potenz gegenüber Problemunkräutern als das aus dem Stand der Technik bekannte N-(2,4-Difluorphenyl)-2-[3-(trifluormethyl)-phenoxy]-3-pyridincarboxamid, welches chemisch und wirkungsmäßig eine naheliegende Verbindung darstellt.

Die erfindungsgemäßen 2-Iminopyridin-Derivate sind durch die Formel (I) allgemein definiert. Bevorzugt sind Verbindungen der Formel (I), bei welchen

| | |
|---|---|
| $R^1$, $R^2$, $R^3$ und $R^4$ | unabhängig voneinander für Wasserstoff, Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, jeweils geradkettiges oder verzweigtes Alkoxy oder Alkylthio mit 1 bis 4 Kohlenstoffatomen, jeweils geradkettiges oder verzweigtes Halogenalkoxy oder Halogenalkylthio mit 1 bis 4 Kohlenstoffatomen und 1 bis 9 gleichen oder verschiedenen Halogenatomen stehen, |
| $R^5$ | für geradkettiges oder verzweigtes Alkylcarbonyl mit 1 bis 20 Kohlenstoffatomen im Alkylteil, für Cycloalkylcarbonyl mit 3 bis 6 Kohlenstoffatomen im Cycloalkylteil für jeweils geradkettiges oder verzweigtes Alkenylcarbonyl oder Alkinylcarbonyl mit jeweils 2 bis 20 Kohlenstoffatomen im Alkenyl- oder Alkinylteil, für geradkettiges oder verzweigtes Halogenalkylcarbonyl mit 1 bis 20 Kohlenstoffatomen im Alkylteil und 1 bis 41 gleichen oder verschiedenen Halogenatomen, für geradkettiges oder verzweigtes Alkoxyalkylcarbonyl mit jeweils 1 bis 4 Kohlenstoffatomen im Alkoxy- und im Alkylteil, für geradkettiges oder verzweigtes Halogenalkenylcarbonyl mit 2 bis 20 Kohlenstoffatomen im Alkenylteil und 1 bis 39 gleichen oder verschiedenen Halogenatomen, für geradkettiges oder verzweigtes Halogenalkinylcarbonyl mit 2 bis 20 Kohlenstoffatomen im Alkinylteil und 1 bis 37 gleichen oder |

verschiedenen Halogenatomen, für geradkettiges oder verzweigtes Alkoxycarbonyl mit 1 bis 20 Kohlenstoffatomen im Alkoxyteil, für geradkettiges oder verzweigtes Alkylcarbonyloxyalkylcarbonyl mit jeweils 1 bis 4 Kohlenstoffatomen in den Alkylteilen, für Alkoxycarbonylcarbonyl mit 1 bis 4 Kohlenstoffatomen im Alkoxyteil, für geradkettiges oder verzweigtes Alkylthiocarbonyl mit 1 bis 20 Kohlenstoffatomen im Alkylthioteil, für jeweils gegebenenfalls einfach oder mehrfach, gleich oder verschieden substituiertes Phenylcarbonyl, Phenylmethylcarbonyl, Phenylethylcarbonyl, Phenylpropylcarbonyl, Phenylethenylcarbonyl, Furylcarbonyl, Thienylcarbonyl, Pyridylcarbonyl, Phenoxymethylcarbonyl, Phenoxy ethylcarbonyl, Phenoxycarbonyl, oder Phenylaminocarbonyl steht, wobei als Substituenten jeweils infrage kommen: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen sowie geradkettiges oder verzweigtes Halogenalkoxy mit 1 bis 4 Kohlenstoffatomen und 1 bis 9 gleichen oder verschiedenen Halogenatomen; $R^5$ weiterhin für geradkettiges oder verzweigtes Alkylsulfonyl mit 1 bis 4 Kohlenstoffatomen, für geradkettiges oder verzweigtes Halogenalkylsulfonyl mit 1 bis 4 Kohlenstoffatomen und 1 bis 9 gleichen oder verschiedenen Halogenatomen, für Alkylaminocarbonyl mit 1 bis 20 Kohlenstoffatomen im geradkettigen oder verzweigten Alkylteil oder für gegebenenfalls einfach oder mehrfach, gleich oder verschieden substituiertes Phenylsulfonyl steht, wobei als Phenylsubstituenten jeweils infrage kommen: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen sowie Alkoxycarbonyl mit 1 bis 4 Kohlenstoffatomen im geradkettigen oder verzweigten Alkoxyteil,

A für geradkettiges oder verzweigtes Alkandiyl mit 1 bis 4 Kohlenstoffatomen steht und

Z für jeweils gegebenenfalls einfach oder mehrfach, gleich oder verschieden substituiertes Phenyl, Pyridinyl, Thienyl, Thiazolyl, Isothiazolyl, Isoxazolyl oder für Oxa-2,4-diazolyl steht, wobei als Substituenten jeweils infrage kommen: Nitro, Cyano, Halogen, geradkettiges oder verzweigtes Alkyl mit 1 bis 4 Kohlenstoffatomen, jeweils geradkettiges oder verzweigtes Halogenalkyl oder Halogenalkylthio mit jeweils 1 bis 4 Kohlenstoffatomen und jeweils 1 bis 9 gleichen oder verschiedenen Halogenatomen, jeweils geradkettiges oder verzweigtes Alkoxy, Alkylthio oder Alkylsulfonyl mit jeweils 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Alkoxycarbonyl mit 1 bis 4 Kohlenstoffatomen im Alkoxyteil sowie gegebenenfalls einfach oder mehrfach, gleich oder verschieden durch Halogen, $C_1$-$C_4$-Alkyl und Halogen-$C_1$-$C_4$-alkyl substituiertes Phenoxy.

Besonders bevorzugt sind Verbindungen der Formel (I), bei welchen

$R^1$ für Wasserstoff steht,

$R^2$ für Wasserstoff, Fluor, Chlor, Brom, Methyl, Ethyl oder Trifluormethyl steht,

$R^3$ für Wasserstoff, Methyl, Ethyl oder Trifluormethyl steht,

$R^4$ für Wasserstoff steht,

$R^5$ für geradkettiges oder verzweigtes Alkylcarbonyl mit 1 bis 10, vorzugsweise 1 bis 5, Kohlenstoffatomen im Alkylteil, für $C_3$-$C_6$-Cycloalkyl-carbonyl, für jeweils geradkettiges oder verzweigtes Alkenylcarbonyl oder Alkinylcarbonyl mit jeweils 2 bis 10, vorzugsweise 2 bis 5, Kohlenstoffatomen im Alkenyl- oder Alkinylteil, für geradkettiges oder verzweigtes Halogenalkylcarbonyl mit 1 bis 10, vorzugsweise 1 bis 4, Kohlenstoffatomen und 1 bis 21, vorzugsweise 1 bis 9, Fluor- und/oder Chloratomen, insbesondere für Trihalogenmethylcarbonyl, für Methoxyacetyl, für Acetyloxyacetyl, für geradkettiges oder verzweigtes Halogenalkenylcarbonyl mit 2 bis 10, vorzugsweise 2 bis 4, Kohlenstoffatomen im Alkenylteil und 1 bis 19, vorzugsweise 1 bis 3 Fluor- und/oder Chloratomen, geradkettiges oder verzweigtes Alkinylcarbonyl mit 2 bis 10, vorzugsweise 2 bis 4,

Kohlenstoffatomen im Alkinylteil und 1 bis 17, vorzugsweise 1 bis 3 Fluor- und oder Chloratomen, für geradkettiges oder verzweigtes Alkoxycarbonyl mit 1 bis 10, vorzugsweise 1 bis 5, Kohlenstoffatomen im Alkoxyteil, für geradkettiges oder verzweigtes Alkylthiocarbonyl mit 1 bis 10, vorzugsweise 1 bis 5, Kohlenstoffatomen im Alkylteil, für jeweils gegebenenfalls ein- oder zweifach, gleich oder verschieden durch Fluor, Chlor, Brom, Cyano, Nitro, Methyl, Trifluormethyl oder Methoxy substituiertes Phenylcarbonyl, Phenylmethylcarbonyl, Phenylethylcarbonyl, Phenylpropylcarbonyl, Phenylethenylcarbonyl, Furylcarbonyl, Thienylcarbonyl, Pyridylcarbonyl, Phenoxymethylcarbonyl, Phenoxyethylcarbonyl, Phenoxycarbonyl oder Phenylaminocarbonyl, für Methylsulfonyl, Ethylsulfonyl, Trifluormethylsulfonyl, für geradkettiges oder verzweigtes Alkylaminocarbonyl mit 1 bis 10, vorzugsweise 1 bis 5, Kohlenstoffatomen im Alkylteil, oder für gegebenenfalls ein- oder zweifach, gleich oder verschieden durch Fluor, Chlor, Cyano, Nitro, Methyl, Trifluormethyl, Methoxycarbonyl oder Ethoxycarbonyl substituiertes Phenylsulfonyl steht,

A für Methandiyl oder Ethandiyl, insbesondere für Methandiyl, steht und

Z für jeweils gegebenenfalls einfach oder zweifach, gleich oder verschieden substituiertes Phenyl, Pyridinyl, Thienyl, Thiazolyl, Isothiazolyl, Isoxazolyl oder Oxa-2,4-diazolyl steht, wobei als Substituenten jeweils infrage kommen: Nitro, Cyano, Fluor, Chlor, Brom, Methyl, Ethyl, Trifluormethyl, Difluormethyl, Methylthio, Methylsulfonyl, Trifluormethylthio, Difluormethylthio, Methoxy, Ethoxy, Trifluormethoxy, Difluormethoxy, Methoxycarbonyl, Ethoxycarbonyl sowie Phenoxy.

Bevorzugt sind die erfindungsgemäß zu verwendenden neuen und bekannten 2-Iminopyridin-Derivate der Formel (IA), bei welchen

$R^1$, $R^2$, $R^3$ und $R^4$ unabhängig voneinander für Wasserstoff, Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, jeweils geradkettiges oder verzweigtes Alkoxy oder Alkylthio mit 1 bis 4 Kohlenstoffatomen oder jeweils geradkettiges oder verzweigtes Halogenalkoxy oder Halogenalkylthio mit 1 bis 4 Kohlenstoffatomen und 1 bis 9 gleichen oder verschiedenen Halogenatomen stehen,

$R^{5-2}$ für Cyano, geradkettiges oder verzweigtes Alkylcarbonyl mit 1 bis 20 Kohlenstoffatomen im Alkylteil, für Cycloalkylcarbonyl mit 3 bis 6 Kohlenstoffatomen im Cycloalkylteil, für jeweils geradkettiges oder verzweigtes Alkenylcarbonyl oder Alkinylcarbonyl mit jeweils 2 bis 20 Kohlenstoffatomen im Alkenyl-oder Alkinylteil, für geradkettiges oder verzweigtes Halogenalkylcarbonyl mit 1 bis 20 Kohlenstoffatomen im Alkylteil und 1 bis 41 gleichen oder verschiedenen Halogenatomen, für geradkettiges oder verzweigtes Alkoxyalkylcarbonyl mit jeweils 1 bis 4 Kohlenstoffatomen im Alkoxy- und im Alkylteil, für geradkettiges oder verzweigtes Halogenalkenylcarbonyl mit 2 bis 20 Kohlenstoffatomen im Alkenylteil und 1 bis 39 gleichen oder verschiedenen Halogenatomen, für geradkettiges oder verzweigtes Halogenalkinylcarbonyl mit 2 bis 20 Kohlenstoffatomen im Alkinylteil und 1 bis 37 gleichen oder verschiedenen Halogenatomen, für geradkettiges oder verzweigtes Alkoxycarbonyl mit 1 bis 20 Kohlenstoffatomen im Alkoxyteil, für geradkettiges oder verzweigtes Alkylcarbonyloxyalkylcarbonyl mit jeweils 1 bis 4 Kohlenstoffatomen in den Alkylteilen, für Alkoxycarbonylcarbonyl mit 1 bis 4 Kohlenstoffatomen im Alkoxyteil, für geradkettiges oder verzweigtes Alkylthiocarbonyl mit 1 bis 20 Kohlenstoffatomen im Alkylteil für jeweils gegebenenfalls einfach oder mehrfach, gleich oder verschieden substituiertes Phenylcarbonyl, Phenylmethylcarbonyl, Phenylethylcarbonyl, Phenylpropylcarbonyl, Phenylethenylcarbonyl, Furylcarbonyl, Thienylcarbonyl, Pyridylcarbonyl, Phenoxymethylcarbonyl, Phenoxyethylcarbonyl, Phenoxycarbonyl oder Phenylaminocarbonyl steht, wobei als Substituenten jeweils infrage kommen: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen sowie geradkettiges oder verzweigtes Halogenalkoxy mit 1 bis 4 Kohlenstoffatomen und 1 bis 9 gleichen oder verschiedenen Halogenatomen; $R^{5-2}$ weiterhin für geradkettiges oder verzweigtes Alkylsulfonyl mit 1 bis 4 Kohlenstoffatomen, für geradkettiges oder verzweigtes Halogenalkylsulfonyl mit 1 bis 4 Kohlenstoffatomen und 1 bis 9 gleichen oder verschiedenen Halogenatomen, für Alkylaminocarbonyl mit 1 bis 20 Kohlenstoffatomen im geradkettigen oder verzweigten Alkylteil oder für

gegebenenfalls einfach oder mehrfach, gleich oder verschieden substituiertes Phenylsulfonyl steht, wobei als Phenylsubstituenten jeweils infrage kommen: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen sowie Alkoxycarbonyl mit 1 bis 4 Kohlenstoffatomen im geradkettigen oder verzweigten Alkoxyteil,

A für geradkettiges oder verzweigtes Alkandiyl mit 1 bis 4 Kohlenstoffatomen steht und

Z für jeweils gegebenenfalls einfach oder mehrfach, gleich oder verschieden substituiertes Phenyl, Pyridinyl, Thienyl, Thiazolyl, Isothiazolyl, Isoxazolyl oder für Oxa-2,4-diazolyl steht, wobei als Substituenten jeweils infrage kommen: Nitro, Cyano, Halogen, geradkettiges oder verzweigtes Alkyl mit 1 bis 4 Kohlenstoffatomen, jeweils geradkettiges oder verzweigtes Halogenalkyl oder Halogenalkylthio mit jeweils 1 bis 4 Kohlenstoffatomen und jeweils 1 bis 9 gleichen oder verschiedenen Halogenatomen, jeweils geradkettiges oder verzweigtes Alkoxy, Alkylthio oder Alkylsulfonyl mit jeweils 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Alkoxycarbonyl mit 1 bis 4 Kohlenstoffatomen im Alkoxyteil sowie gegebenenfalls einfach oder mehrfach, gleich oder verschieden durch Halogen, $C_1$-$C_4$-Alkyl oder Halogen-$C_1$-$C_4$-alkyl substituiertes Phenoxy.

Besonders bevorzugt sind die erfindungsgemäß zu verwendenden Verbindungen der Formel (1A), bei welchen

$R^1$ für Wasserstoff steht,

$R^2$ für Wasserstoff, Fluor, Chlor, Brom, Methyl, Ethyl oder Trifluormethyl steht,

$R^3$ für Wasserstoff, Methyl, Ethyl oder Trifluormethyl steht,

$R^4$ für Wasserstoff steht,

$R^{5-2}$ für Cyano, geradkettiges oder verzweigtes Alkylcarbonyl mit 1 bis 10, vorzugsweise 1 bis 5, Kohlenstoffatomen im Alkylteil, für $C_3$-$C_6$-Cycloalkylcarbonyl, für jeweils geradkettiges oder verzweigtes Alkenylcarbonyl oder Alkinylcarbonyl mit jeweils 2 bis 10, vorzugsweise 2 bis 5, Kohlenstoffatomen im Alkenyl-oder Alkinylteil, für geradkettiges oder verzweigtes Halogenalkylcarbonyl mit 1 bis 10, vorzugsweise 1 bis 4, Kohlenstoffatomen und 1 bis 21, vorzugsweise 1 bis 9 Fluor- und/oder Chloratomen, für Methoxyacetyl, für Acetyloxyacetyl, für gerad kettiges oder verzweigtes Halogenalkenylcarbonyl mit 2 bis 10, vorzugsweise 2 bis 4, Kohlenstoffatomen im Alkenylteil und 1 bis 19, vorzugsweise 1 bis 3 Fluor- und/oder Chloratomen, geradkettiges oder verzweigtes Alkinylcarbonyl mit 2 bis 10, vorzugsweise 2 bis 4, Kohlenstoffatomen im Alkinylteil und 1 bis 17, vorzugsweise 1 bis 3 Fluor- und/oder Chloratomen, für geradkettiges oder verzweigtes Alkoxycarbonyl mit 1 bis 10, vorzugsweise 1 bis 5, Kohlenstoffatomen im Alkoxyteil, für geradkettiges oder verzweigtes Alkylthiocarbonyl mit 1 bis 10, vorzugsweise 1 bis 5, Kohlenstoffatomen im Alkylteil, für jeweils gegebenenfalls ein- oder zweifach, gleich oder verschieden durch Fluor, Chlor, Brom, Cyano, Nitro, Methyl, Trifluormethyl oder Methoxy substituiertes Phenylcarbonyl, Phenylmethylcarbonyl, Phenylethylcarbonyl, Phenylpropylcarbonyl, Phenylethenylcarbonyl, Furylcarbonyl, Thienylcarbonyl, Pyridylcarbonyl, Phenoxymethylcarbonyl, Phenoxycarbonyl oder Phenylaminocarbonyl, für Methylsulfonyl, Ethylsulfonyl, Trifluormethylsulfonyl, für geradkettiges oder verzweigtes Alkylaminocarbonyl mit 1 bis 10, vorzugsweise 1 bis 5, Kohlenstoffatomen im Alkylteil, oder für gegebenenfalls ein- oder zweifach, gleich oder verschieden durch Fluor, Chlor, Cyano, Nitro, Methyl, Trifluormethyl, Methoxycarbonyl oder Ethoxycarbonyl substituiertes Phenylsulfonyl steht,

A für Methandiyl oder Ethandiyl, insbesondere Methandiyl, steht und

Z für jeweils gegebenenfalls einfach oder zweifach, gleich oder verschieden substituiertes Phenyl, Pyridinyl, Thienyl, Thiazolyl, Isothiazolyl, Isoxazolyl oder Oxa-2,4-diazolyl steht, wobei als Substituenten jeweils infrage kommen: Nitro, Cyano, Fluor, Chlor, Brom, Methyl, Ethyl, Trifluormethyl, Difluormethyl, Methylthio, Methylsulfonyl, Trifluormethylthio, Difluormethylthio, Methoxy, Ethoxy, Trifluormethoxy, Difluormethoxy, Methoxycarbonyl, Ethoxycarbonyl sowie Phenoxy.

Verwendet man beispielsweise N-[(3-Trifluormethyl)-benzyl]-5-methyl-2-iminopyridin-hydrochlorid und

Chlorameisensäureethylester als Ausgangsstoffe und Triethylamin als Base, so läßt sich der Reaktionsablauf des erfindungsgemäßen Verfahrens (a) durch das folgende Formelschema darstellen:

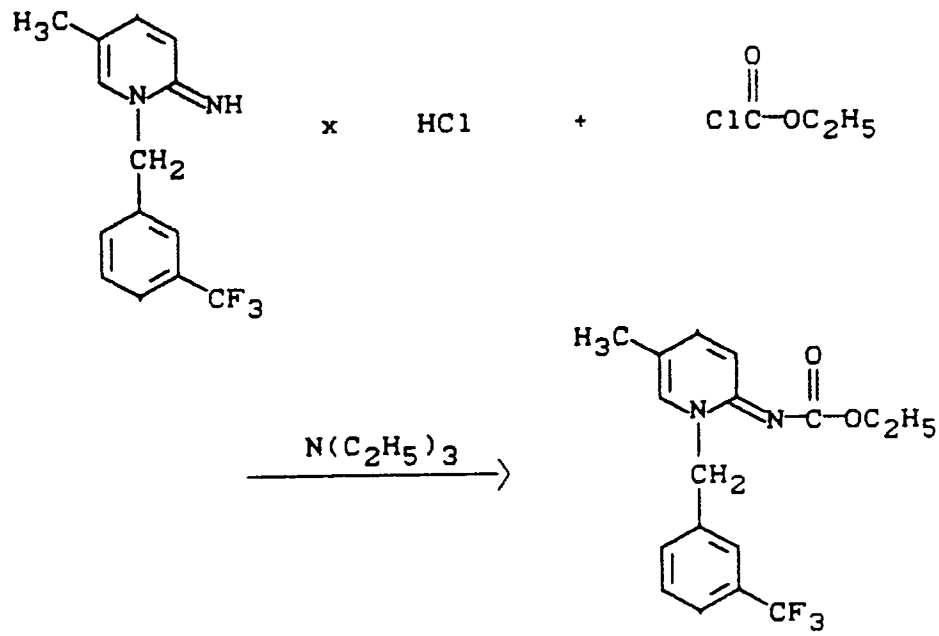


Verwendet man beispielsweise N-[(3-Trifluormethyl)-benzyl]-5-methyl-2-iminopyridin-hydrochlorid und Methylisocyanat, so läßt sich das erfindungsgemäße Verfahren (b) durch das folgende Formelschema darstellen:

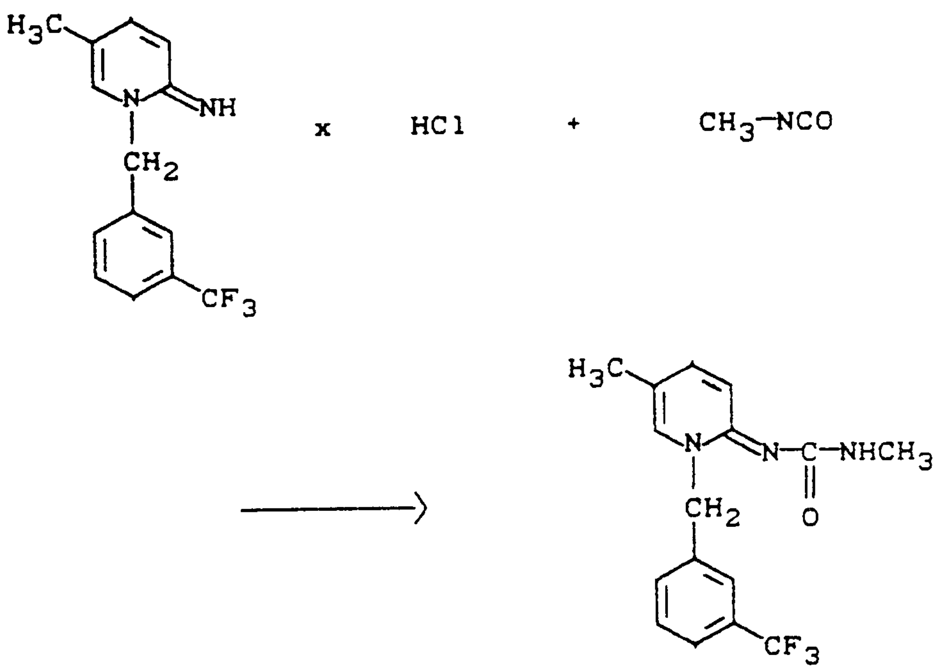


Die bei den erfindungsgemäßen Verfahren (a) und (b) zur Herstellung von Verbindungen der Formel (I) als Ausgangsstoffe zu verwendenden 1,2-Dihydropyridin-iminiumsalze sind durch die Formel (II) allgemein definiert.

In Formel (II) haben $R^1$, $R^2$, $R^3$, $R^4$, A und Z vorzugsweise bzw. insbesondere diejenigen Bedeutungen, die bereits oben im Zusammenhang mit der Beschreibung der erfindungsgemäßen Verbindungen der Formel (I), vorzugsweise bzw. als insbesondere bevorzugt für $R^1$, $R^2$, $R^3$, $R^4$, A und Z, angegeben wurden.

HX steht vorzugsweise für das Äquivalent einer Mineralsäure, wie z.B. Chlorwasserstoffsäure oder Bromwasserstoffsäure oder einer Carbonsäure, wie z.B. Oxalsäure.

Beispiele für Ausgangsstoffe der Formel (II) sind in Tabelle 1 aufgeführt.

**Tabelle 1**

| $R^1$ | $R^2$ | $R^3$ | $R^4$ | A | Z | HX |
|---|---|---|---|---|---|---|
| H | $CH_3$ | H | H | $-CH_2-$ | 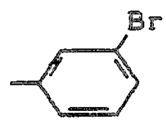 | HBr |
| H | Cl | H | H | $-CH_2-$ | 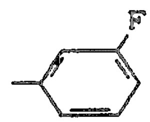 | HCl |
| H | Br | H | H | $-CH_2-$ | 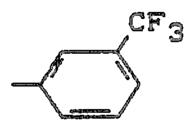 | HCl |
| H | $CH_3$ | H | H | $-CH_2-$ | 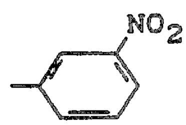 | HCl |
| H | $CH_3$ | H | H | $-CH_2-$ | 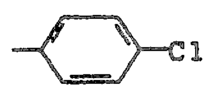 | HCl |
| H | $CH_3$ | H | H | $-CH_2-$ | 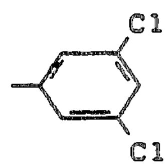 | HCl |

Tabelle 1 (Fortsetzung)

| $R^1$ | $R^2$ | $R^3$ | $R^4$ | A | Z | HX |
|---|---|---|---|---|---|---|
| H | F | $CH_3$ | H | $-CH_2-$ | 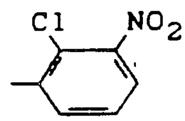 | HBr |
| H | $C_2H_5$ | $CH_3$ | H | $-CH_2-$ | 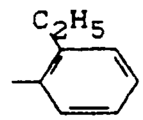 | HBr |
| H | $CF_3$ | $CH_3$ | H | $-CH_2-$ | 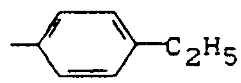 | HCl |
| H | $CF_3$ | $C_2H_5$ | H | $-CH_2-$ | 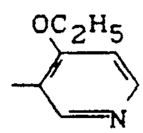 | HCl |
| H | $C_2H_5$ | $C_2H_5$ | H | $-CH_2-$ | 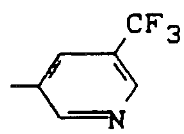 | HBr |
| H | F | $C_2H_5$ | H | $-CH_2-$ | 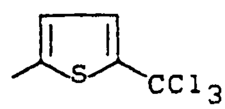 | HCl |
| H | F | $CF_3$ | H | $-CH_2-$ | 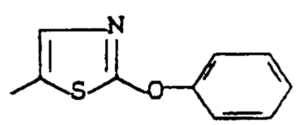 | HCl |
| H | $C_2H_5$ | $CF_3$ | H | $-CH_2-$ | 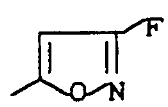 | HCl |
| H | $CH_3$ | $CF_3$ | H | $-CH_2-$ | 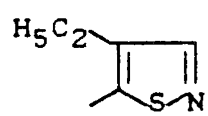 | HBr |

<u>Tabelle 1</u> (Fortsetzung)

| $R^1$ | $R^2$ | $R^3$ | $R^4$ | A | Z | HX |
|---|---|---|---|---|---|---|
| H | Cl | $CF_3$ | H | $-CH_2-$ | 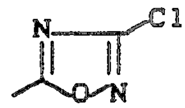 | HBr |
| H | $CF_3$ | $CF_3$ | H | $-CH_2-$ | 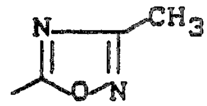 | HCl |
| H | $C_2H_5$ | H | H | $-CH_2-$ | 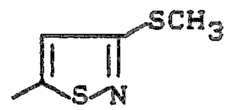 | HCl |
| H | $C_2H_5$ | $CH_3$ | H | $-CH_2-$ | 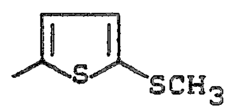 | HBr |
| H | H | $CF_3$ | H | $-CH_2-$ | 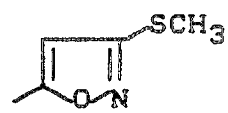 | HCl |

Die 1,2-Dihydropyridin-iminiumsalze der Formel (II) sind teilweise bekannt (vergleiche US 3 933 836; J. Heterocycl. Chem., 12(5), 1027 - 1029) und/oder können nach an sich bekannten Verfahren hergestellt werden, indem man 2-Aminopyridin-Derivate der allgemeinen Formel (V),

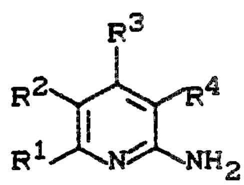 (V)

in welcher
$R^1$, $R^2$, $R^3$ und $R^4$ die oben angegebene Bedeutung haben,
mit Verbindungen der Formel (VI),

$$X-A-Z \qquad (VI)$$

in welcher
A und Z die oben angegebene Bedeutung haben und
X für Halogen, vorzugsweise für Chlor oder Brom steht,
gegebenenfalls in Gegenwart eines Verdünnungsmittels wie beispielsweise Acetonitril, bei Temperaturen zwischen 0° C und 140° C umsetzt.

2-Aminopyridin-Derivate der Formel (V) und Verbindungen der Formel (VI) sind allgemein bekannte Verbindungen der organischen Chemie.

Die beim erfindungsgemäßen Verfahren (a) zur Herstellung von Verbindungen der Formel (I) außerdem als Ausgangsstoffe zu verwendenden Halogenverbindungen sind durch die Formel (III) allgemein definiert.

In Formel (III) steht $R^{5-1}$ vorzugsweise für geradkettiges oder verzweigtes Alkylcarbonyl mit 1 bis 20

Kohlenstoffatomen im Alkylteil, für Cycloalkylcarbonyl mit 3 bis 6 Kohlenstoffatomen im Cycloalkylteil, für jeweils geradkettiges oder verzweigtes Alkenylcarbonyl oder Alkinylcarbonyl mit jeweils 2 bis 20 Kohlenstoffatomen im Alkenyl- oder Alkinylteil, für geradkettiges oder verzweigtes Halogenalkylcarbonyl mit 2 bis 20 Kohlenstoffatomen im Alkylteil und 1 bis 41 gleichen oder verschiedenen Halogenatomen, für geradkettiges oder verzweigtes Alkoxyalkylcarbonyl mit jeweils 1 bis 4 Kohlenstoffatomen im Alkoxy- und im Alkylteil, für geradkettiges oder verzweigtes Halogenalkenylcarbonyl mit 2 bis 20 Kohlenstoffatomen im Alkenylteil und 1 bis 39 gleichen oder verschiedenen Halogenatomen, für geradkettiges oder verzweigtes Halogenalkinylcarbonyl mit 2 bis 20 Kohlenstoffatomen im Alkinylteil und 1 bis 37 gleichen oder verschiedenen Halogenatomen, für geradkettiges oder verzweigtes Alkoxycarbonyl mit 1 bis 20 Kohlenstoffatomen im Alkoxyteil, für geradkettiges oder verzweigtes Alkylcarbonyloxyalkylcarbonyl mit jeweils 1 bis 4 Kohlenstoffatomen in den Alkylteilen, für Alkoxycarbonylcarbo nyl mit 1 bis 4 Kohlenstoffatomen im Alkoxyteil, für geradkettiges oder verzweigtes Alkylthiocarbonyl mit 1 bis 20 Kohlenstoffatomen im Alkylteil, für jeweils gegebenenfalls einfach oder mehrfach, gleich oder verschieden substituiertes Phenylcarbonyl, Phenylmethylcarbonyl, Phenylethylcarbonyl, Phenylpropylcarbonyl, Phenylethenylcarbonyl, Furylcarbonyl, Thienylcarbonyl, Pyridylcarbonyl, Phenoxymethylcarbonyl, oder Phenoxycarbonyl, wobei als Substituenten jeweils infrage kommen: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen sowie geradkettiges oder verzweigtes Halogenalkoxy mit 1 bis 4 Kohlenstoffatomen und 1 bis 9 gleichen oder verschiedenen Halogenatomen; weiterhin für geradkettiges oder verzweigtes Alkylsulfonyl mit 1 bis 4 Kohlenstoffatomen, für geradkettiges oder verzweigtes Halogenalkylsulfonyl mit 1 bis 4 Kohlenstoffatomen und 1 bis 9 gleichen oder verschiedenen Halogenatomen, oder für gegebenenfalls einfach oder mehrfach, gleich oder verschieden substituiertes Phenylsulfonyl, wobei als Phenylsubstituenten infrage kommen: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen sowie Alkoxycarbonyl mit 1 bis 4 Kohlen stoffatomen im geradkettigen oder verzweigten Alkoxyteil.

Besonders bevorzugt steht $R^{5-1}$ für geradkettiges oder verzweigtes Alkylcarbonyl mit 2 bis 10 Kohlenstoffatomen im Alkylteil, für $C_3$-$C_6$-Cycloalkylcarbonyl, für jeweils geradkettiges oder verzweigtes Alkenylcarbonyl oder Alkinylcarbonyl mit jeweils 2 bis 10 Kohlenstoffatomen im Alkenyl- oder Alkinylteil, für geradkettiges oder verzweigtes Halogenalkylcarbonyl mit 1 bis 10 Kohlenstoffatomen und 1 bis 21 Fluor- und/oder Chloratomen, für Methoxyacetyl, für Acetyloxyacetyl, für geradkettiges oder verzweigtes Halogenalkenylcarbonyl mit 2 bis 10 Kohlenstoffatomen im Alkenylteil und 1 bis 19 Fluor- und oder Chloratomen, geradkettiges oder verzweigtes Halogenalkinylcarbonyl mit 2 bis 10 Kohlenstoffatomen im Alkinylteil und 1 bis 17 Fluor- und/oder Chloratomen, für geradkettiges oder verzweigtes Alkoxycarbonyl mit 1 bis 10 Kohlenstoffatomen im Alkoxyteil, für geradkettiges oder verzweigtes Alkylthiocarbonyl mit 1 bis 10 Kohlenstoffatomen im Alkylteil, für jeweils gegebenenfalls ein- oder zweifach, gleich oder verschieden durch Fluor, Chlor, Brom, Cyano, Nitro, Methyl, Trifluormethyl oder Methoxy substituiertes Phenylcarbonyl, Phenylmethylcarbonyl, Phenylethylcarbonyl, Phenylpropylcarbonyl, Phenylethenylcarbonyl, Furylcarbonyl, Thienylcarbonyl, Pyridylcarbonyl, Phenoxymethylcarbonyl, oder Phenoxycarbonyl, für Methylsulfonyl, Ethylsulfonyl, Trifluormethylsulfonyl oder für gegebenenfalls ein- oder zweifach, gleich oder verschieden durch Fluor, Chlor, Cyano, Nitro, Methyl, Trifluormethyl, Methoxycarbonyl oder Ethoxycarbonyl substituiertes Phenylsulfonyl.

X steht vorzugsweise für Chlor oder Brom, besonders bevorzugt für Chlor.

Als Beispiele für die Ausgangsstoffe der Formel (III) seien genannt: Acetylchlorid, Pivaloylchlorid, n-Dodecansäurechlorid, Acrylsäurechlorid, Methacrylsäurechlorid, Crotonsäurechlorid, Vinylessigsäurechlorid, 9-Dodecensäurechlorid, Propinsäurechlorid, Difluoracetylchlorid, 2-Chlorpropionylchlorid, Chlorameisensäureethylester, 4-Chlor-benzoylchlorid, Chlorameisensäure-(3-nitrophenyl)ester, Methylsulfonylchlorid, 3-Ethoxycarbonyl-phenylsulfonylchlorid und - für den Fall, daß $R^{5-1}$ für Alkylcarbonyl, Alkenylcarbonyl, Alkinylcarbonyl, Halogenalkylcarbonyl, Halogenalkenylcarbonyl, Halogenalkinylcarbonyl oder für gegebenenfalls substituiertes Phenylcarbonyl steht - seien beispielhaft genannt: Essigsäureanhydrid, Acrylsäureanhydrid, 3-Chloracrylsäureanhydrid, Propinsäureanhydrid und Trifluoressigsäureanhydrid.

Das erfindungsgemäße Verfahren (a) zur Herstellung der neuen 2-Iminopyridin-Derivate der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan,

Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Gly koldimethylether und Diglykoldimethylether, Tetrahydrofuran und Dioxan, Ketone wie Aceton, Methyl-ethyl-, Methylisopropyl-und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Als Basen kommen für das erfindungsgemäße Verfahren (a) alle üblicherweise verwendbaren anorganischen und organischen Basen in Betracht. Vorzugsweise verwendbar sind Alkalimetallcarbonate, wie z.B. Natrium- und Kaliumcarbonat; Alkalimetallhydroxide, wie z.B. Natriumhydroxid; Alkalimetallalkoholate, wie z.B. Natrium- und Kaliummethylat und -ethylat; Alkalimetallhydride, wie z.B. Natriumhydrid; niedere tertiäre Alkylamine, Cycloalkylamine und Aralkylamine, wie insbesondere Triethylamin, sowie Alkalimetallalkyl-Verbindungen wie z.B. n-Butyllithium.

Die Reaktionstemperaturen können bei dem erfindungsgemäßen Verfahren (a) in einem größeren Bereich variiert werden. Im allgemeinen arbeitet man bei Temperaturen zwischen 0 °C und +200 °C, vorzugsweise bei Temperaturen zwischen +20 °C und +100 °C.

Zur Durchführung des erfindungsgemäßen Verfahrens (a) setzt man pro Mol an 1,2-Dihydropyridiniminiumsalz der Formel (II) im allgemeinen 1 bis 1,5 Mol, vorzugsweise 1 Mol an Halogenverbindung der Formel (III) sowie im allgemeinen 1 bis 5 Mol, vorzugsweise 1 bis 3 Mol an Base ein.

Die beim erfindungsgemäßen Verfahren (b) zur Herstellung von Verbindungen der Formel (I) als Ausgangsstoffe zu verwendenden Isocyanate sind durch die Formel (IV) allgemein definiert.

In Formel (IV) steht $R^6$ vorzugsweise für geradkettiges oder verzweigtes Alkyl mit 1 bis 20 Kohlenstoffatomen oder für gegebenenfalls einfach oder mehrfach, gleich oder verschieden substituiertes Phenyl, wobei als Substituenten genannt seien: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen.

Besonders bevorzugt steht $R^6$ für geradkettiges oder verzweigtes Alkyl mit 1 bis 10 Kohlenstoffatomen oder gegebenenfalls ein- oder zweifach, gleich oder verschieden durch Fluor, Chor, Cyano, Nitro, Methyl oder Trifluormethyl substituiertes Phenyl.

Als Beispiele für die Ausgangsstoffe der Formel (IV) seien genannt: Methylisocyanat, Ethylisocyanat, n-Propylisocyanat, n-Butylisocyanat, Phenylisocyanat, 2-Chlorphenylisocyanat, 2-Cyanophenylisocyanat, 2-Trifluormethylphenylisocyanat, 2-Methoxycarbonylphenylisocyanat, 3-Nitrophenylisocyanat, 3-Methylphenylisocyanat, 3-Ethoxycarbonylphenylisocyanat, 4-Fluorphenylisocyanat, 4-Trifluormethylphenylisocyanat, 4-Nitrophenylisocyanat sowie 4-Cyanophenylisocyanat.

Die Isocyanate der Formel (IV) sind allgemein bekannte Verbindungen der organischen Chemie.

Das erfindungsgemäße Verfahren (b) zur Herstellung der neuen 2-Iminopyridin-Derivate der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl-und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Als Basen kommen für das erfindungsgemäße Verfahren (b) alle üblicherweise verwendbaren anorganischen und organischen Basen in Betracht. Vorzugsweise verwendbar sind Alkalimetallcarbonate, wie z.B. Natrium- und Kaliumcarbonat; Alkalimetallhydroxide, wie z.B. Natriumhydroxid; Alkalimetallalkoholate, wie z.B. Natrium- und Kaliummethylat und -ethylat; Alkalimetallhydride, wie z.B. Natriumhydrid; sowie niedere tertiäre Alkylamine, Cycloalkylamine und Aralkylamine, wie insbesondere Triethylamin.

Die Reaktionstemperaturen können bei dem erfindungsgemäßen Verfahren (b) in einem größeren Bereich variiert werden. Im allgemeinen arbeitet man bei Temperaturen zwischen 0 °C und 200 °C, vorzugsweise bei Temperaturen zwischen 20 °C und 120 °C.

Zur Durchführung des erfindungsgemäßen Verfahrens (b) setzt man pro Mol an 1,2-Dihydropyridiniminiumsalz der Formel (II) im allgemeinen 1 bis 3 Mol, vorzugsweise 1 bis 1,5 Mol an Isocyanat der Formel (IV) sowie im allgemeinen 3 bis 5 Mol, vorzugsweise 2 bis 2,5 Mol an Base ein.

Die erfindungsgemäßen Wirkstoffe der Formel (I) bzw. (IA) können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe der Formel (I) bzw. (IA) können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der Wirkstoffe der Formeln (I) bzw. (IA) ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weichflächen und zur selektiven Unkrautbekämpfung in einjährigen Kulturen eingesetzt werden.

Dabei eignen sich die erfindungsgemäßen Wirkstoffe der Formel (I) bzw. (IA) besonders gut zur selektiven Bekämpfung von mono-und dikotylen Unkräutern in monokotylen Kulturen im Vor-und Nachauflaufverfahren. Darüber hinaus besitzen einige der Wirkstoffe auch blattinsektizide sowie bodeninsektizide und wurzelsystemische Wirkungseigenschaften.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen,

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel kön nen z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z,B, nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol,

Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden,

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methyl thio-1,2,4-triazin-5(4H)-on (METRIBUZIN) zur Unkrautbekämpfung in Sojabohnen, in Frage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N, N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl- oder deren Ethylester (DICLOFOPMETHYL); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methyl-heptylester (FLUROXYPYR); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl)-benzoesäure oder deren Methylester (METSULFURON); (2-Chlor-4-trifluormethylphenyl)-(3-ethoxy-4-nitrophenyl)-ether (OXYFLUORFEN); N-(1-Ethylpropyl)-3,4-dimethyl-2-6-dinitroanilin (PENDIMETHALIN); 2-Chlor-N-isopropylacetanilid (PROPACHLOR); 0-(6-Chlor-3-phenyl-pyridazin-4-yl)-S-octyl-thiocarbonat (PYRIDATE); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE) und 2,6-Dinitro-4-trifluormethyl-N,N-dipropylanilin (TRIFLURALIN) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösun gen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro ha.

Die Herstellung und die Verwendung der erfindungsgemäßen Wirkstoffe geht aus den nachfolgenden

Beispielen hervor.

Herstellungsbeispiele

Beispiel 1

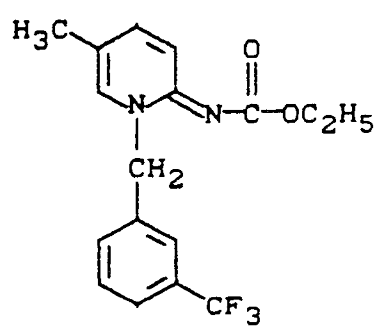

(Verfahren (a))

3,0 g (0,01 Mol) N-[(3-Trifluormethyl)benzyl]-5-methyl-2-iminopyridin-hydrochlorid werden in 100 ml Acetonitril gelöst und mit 2 ml Triethylamin (0,027 Mol) und 1,1 g (0,01 Mol) Chlorameisensäureethylester versetzt. Man rührt die Mischung 18 Stunden bei Raumtemperatur und engt anschließend stark ein. Der ölige Rückstand wird mit Chloroform und Wasser geschüttelt. Die organische Phase wird abgetrennt und durch Verreiben mit Methylcyclohexan kristallisiert. Man erhält 0,7 g (21 % der Theorie) an 2-Ethoxycarbonylimino-5-methyl-1-(3-trifluormethyl-benzyl)-1,2-dihydropyridin vom Schmelzpunkt 121 °C.

Beispiel 2

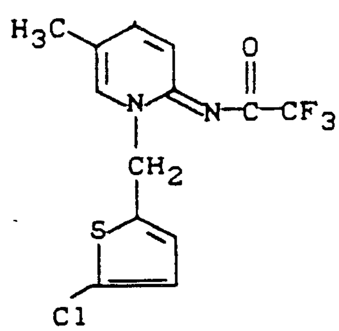

(Verfahren (a))

1,4 g (0,005 Mol) N-(5-Chlorthien-2-yl-methyl)-5-methyl-2-iminopyridin-hydrochlorid werden in 50 ml Acetonitril gelöst und mit 1 ml (0,013 Mol) Triethylamin und 1,1 g (0,005 Mol) Trifluoressigsäureanhydrid versetzt. Man rührt 18 Stunden bei Raumtemperatur und erhitzt anschließend 4 Stunden am Rückfluß. Die Reaktionsmischung wird stark eingeengt, mit Chloroform aufgenommen und über Natriumsulfat getrocknet. Nach Filtration und destillativer Entfernung des Lösungsmittels wird der erhaltene Rückstand durch Verreiben mit Methylcyclohexan kristallisiert.

Man erhält 1,5 g (90 % der Theorie) an 1-(5-Chlorthien-2-yl-methyl)-5-methyl-2-trifluormethylcarbonylimino-1,2-dihydropyridin vom Schmelzpunkt 170 °C.

Analog zu den Beispielen 1 und 2 und gemäß den allgemeinen Angaben zu den erfindungsgemäßen Verfahren (a) und (b) können die in Tabelle 2 aufgeführten erfindungsgemäßen Verbindungen der Formel (I) bzw. (IA) hergestellt werden.

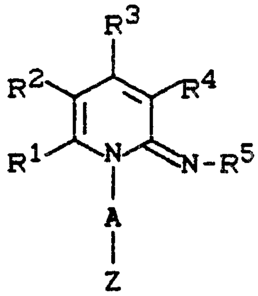 (I) bzw. 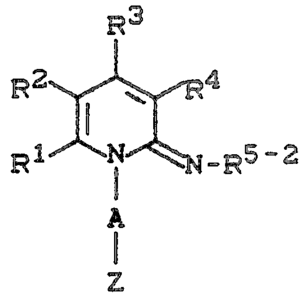 (IA)

Tabelle 2

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 3 | H | $-CH_3$ | H | H | 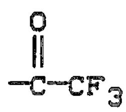 | $-CH_2-$ | 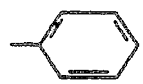 | δ=5,59 |
| 4 | H | $-CH_3$ | H | H | 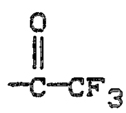 | $-CH_2-$ | 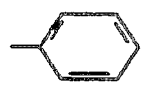 | δ=5,56 |
| 5 | H | H | H | H | 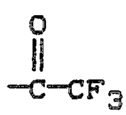 | $-CH_2-$ | 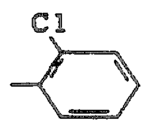 | 90° C |
| 6 | H | H | H | H | 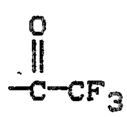 | $-CH_2-$ | 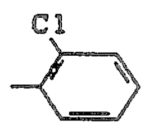 | δ=5,53 |
| 7 | H | H | H | H | $-SO_2-CH_3$ | $-CH_2-$ | 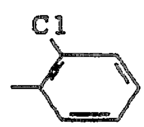 | δ=5,49 |
| 8 | H | H | H | H | 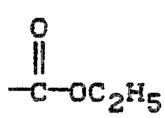 | $-CH_2-$ | 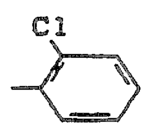 | δ=5,48 (DMSO) |

<u>Tabelle 2</u> (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 9 | H | $-CH_3$ | H | H | 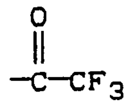 | $-CH_2-$ | 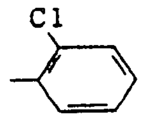 | 140° C |
| 10 | H | $-CH_3$ | H | H | 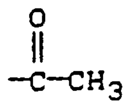 | $-CH_2-$ | 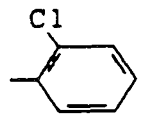 | 98° C |
| 11 | H | $-CH_3$ | H | H | 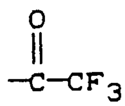 | $-CH_2-$ | 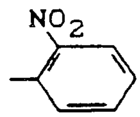 | 168° C |
| 12 | H | $-CH_3$ | H | H | 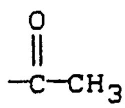 | $-CH_2-$ | 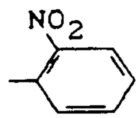 | 157° C |
| 13 | H | $-CH_3$ | H | H | 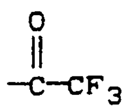 | $-CH_2-$ | 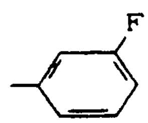 | $\delta$=5,55 |
| 14 | H | $-CH_3$ | H | H | 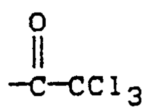 | $-CH_2-$ | 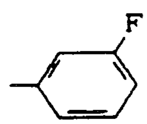 | 153° C |
| 15 | H | $-CH_3$ | H | H | 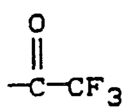 | $-CH_2-$ | 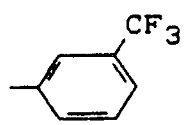 | 129° C |
| 16 | H | $-CH_3$ | H | H | 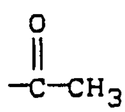 | $-CH_2-$ | 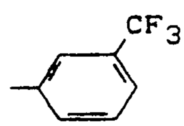 | $\delta$=5,44 |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1$H-NMR* |
|---|---|---|---|---|---|---|---|---|
| 17 | H | $-CH_3$ | H | H | 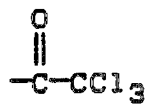 | $-CH_2-$ | 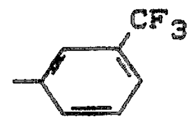 | 198° C |
| 18 | H | $-CH_3$ | H | H | 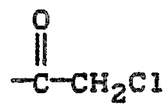 | $-CH_2-$ | 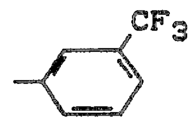 | δ=5,54 |
| 19 | H | $-CH_3$ | H | H | 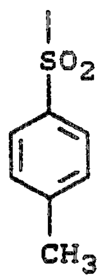 | $-CH_2-$ | 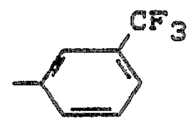 | δ=5,37 |
| 20 | H | $-CH_3$ | H | H | 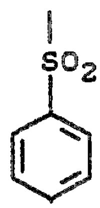 | $-CH_2-$ | 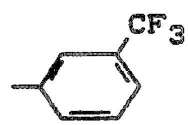 | δ=5,37 |
| 21 | H | $-CH_3$ | H | H | 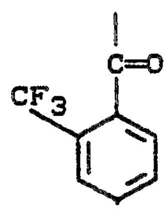 | $-CH_2-$ | 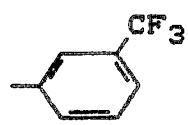 | 148° C |
| 22 | H | $-CH_3$ | H | H | 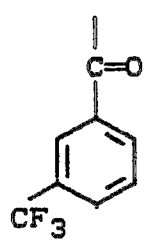 | $-CH_2-$ | 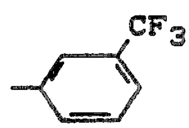 | 134° C |

<u>Tabelle 2</u> (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 23 | H | $-CH_3$ | H | H | 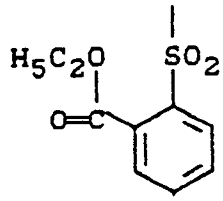 | $-CH_2-$ | 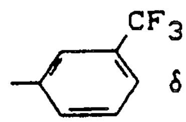 | δ=5,98 |
| 24 | H | $-CH_3$ | H | H | $-CN$ | $-CH_2-$ | 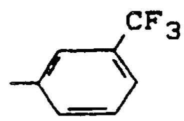 | 168° C |
| 25 | H | $-CH_3$ | H | H | 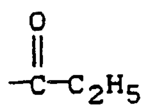 | $-CH_2-$ | 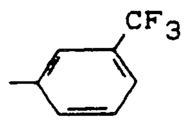 | δ=5,50 |
| 26 | H | $-CH_3$ | H | H | 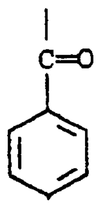 | $-CH_2-$ | 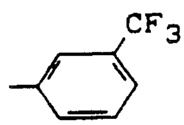 | δ=5,67 |
| 27 | H | $-CH_3$ | H | H | 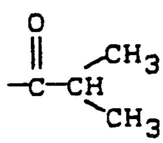 | $-CH_2-$ | 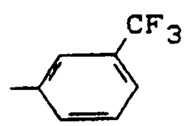 | δ=5,44 |
| 28 | H | $-CH_3$ | H | H | 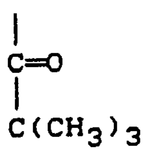 | $-CH_2-$ | 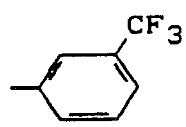 | 109° C |
| 29 | H | H | H | H | 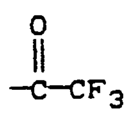 | $-CH_2-$ | 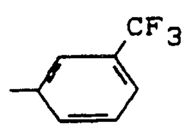 | 125° C |

<u>Tabelle 2</u> (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1$H-NMR* |
|---|---|---|---|---|---|---|---|---|
| 30 | H | H | H | H | 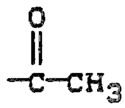 | $-CH_2-$ | 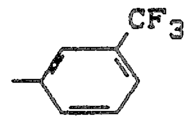 | 92° C |
| 31 | H | H | H | H | 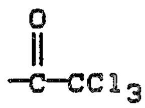 | $-CH_2-$ | 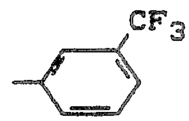 | 95° C |
| 32 | H | H | H | H | $-CN$ | $-CH_2-$ | 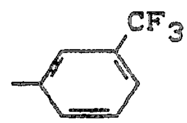 | 152° C |
| 33 | H | Cl | H | H | 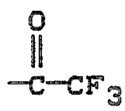 | $-CH_2-$ | 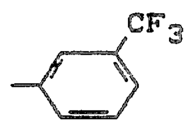 | 134° C |
| 34 | H | Cl | H | H | 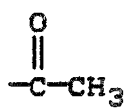 | $-CH_2-$ | 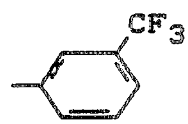 | 128° C |
| 35 | H | Cl | H | H | 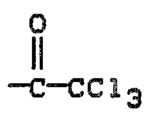 | $-CH_2-$ | 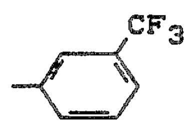 | δ=5,65 (DMSO) |
| 36 | H | H | $-CH_3$ | H | 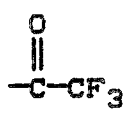 | $-CH_2-$ | 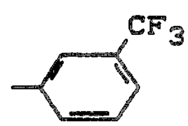 | 132° C |
| 37 | H | H | $-CH_3$ | H | 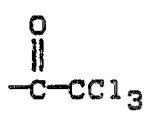 | $-CH_2-$ | 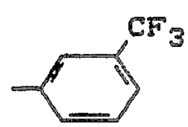 | 158° C |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H-NMR$* |
|---|---|---|---|---|---|---|---|---|
| 38 | H | H | $-CH_3$ | H | 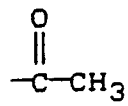 | $-CH_2-$ | 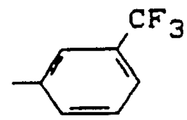 | δ=5,47 (DMSO) |
| 39 | H | H | H | H | 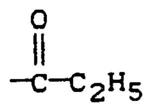 | $-CH_2-$ | 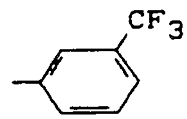 | 121° C |
| 40 | H | Cl | H | H | 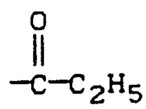 | $-CH_2-$ | 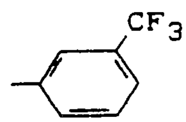 | 100° C |
| 41 | H | $-CH_3$ | H | H | 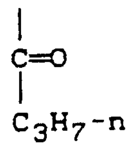 | $-CH_2-$ | 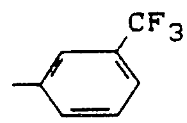 | 117° C |
| 42 | H | $-CH_3$ | H | H | 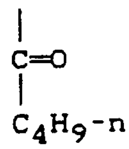 | $-CH_2-$ | 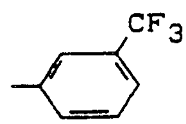 | 78° C |
| 43 | H | $-CH_3$ | H | H | 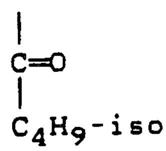 | $-CH_2-$ | 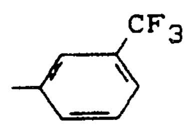 | 95° C |
| 44 | H | H | H | H | 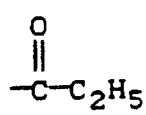 | $-CH_2-$ | 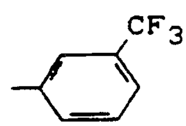 | 84° C |
| 45 | H | $-CH_3$ | H | H | 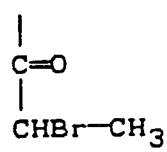 | $-CH_2-$ | 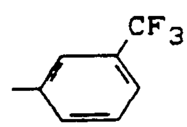 | 85° C |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 46 | H | H | $-CH_3$ | H | 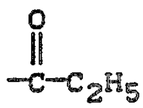 | $-CH_2-$ | 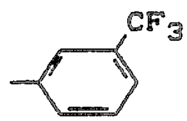 | 152° C |
| 47 | H | Cl | H | H | 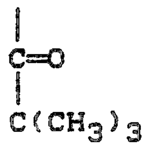 | $-CH_2-$ | 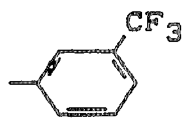 | 92° C |
| 48 | H | H | H | H | 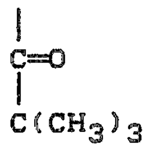 | $-CH_2-$ | 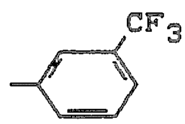 | 138° C |
| 49 | H | H | H | H | 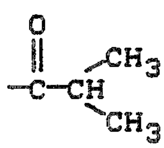 | $-CH_2-$ | 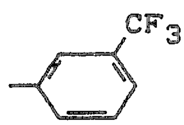 | 98° C |
| 50 | H | $-CH_3$ | H | H | 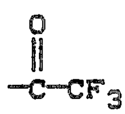 | $-CH_2-$ | 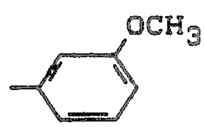 | δ=5,53 |
| 51 | H | $-CH_3$ | H | H | 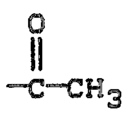 | $-CH_2-$ | 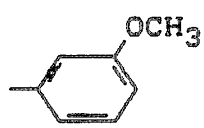 | δ=5,49 |
| 52 | H | $-CH_3$ | H | H | 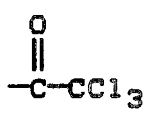 | $-CH_2-$ | 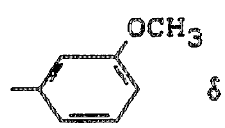 | δ=5,59 |
| 53 | H | $-CH_3$ | H | H | 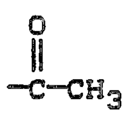 | $-CH_2-$ | 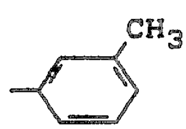 | δ=5,42 |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 54 | H | $-CH_3$ | H | H | 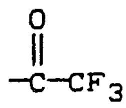 | $-CH_2-$ | 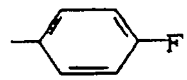 | δ=5,52 |
| 55 | H | $-CH_3$ | H | H | 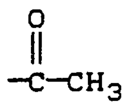 | $-CH_2-$ | 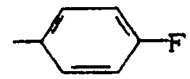 | 130° C |
| 56 | H | $-CH_3$ | H | H | 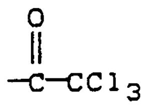 | $-CH_2-$ | 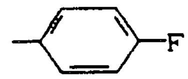 | δ=5,58 |
| 57 | H | $-CH_3$ | H | H | 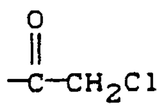 | $-CH_2-$ | 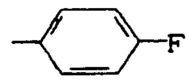 | δ=5,50 |
| 58 | H | $-CH_3$ | H | H | $-CN$ | $-CH_2-$ | 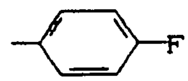 | 226° C |
| 59 | H | H | H | H | 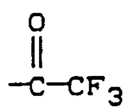 | $-CH_2-$ | 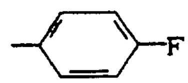 | 138° C |
| 60 | H | H | H | H | 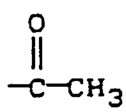 | $-CH_2-$ | 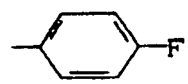 | 93° C |
| 61 | H | H | H | H | 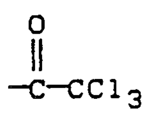 | $-CH_2-$ | 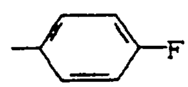 | δ=5,58 |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 62 | H | Cl | H | H | 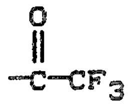 | $-CH_2-$ | 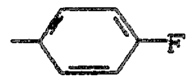 | 149° C |
| 63 | H | Cl | H | H | 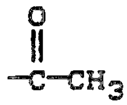 | $-CH_2-$ | 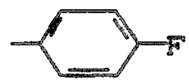 | δ=5,35 |
| 64 | H | Cl | H | H | 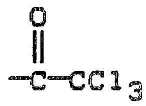 | $-CH_2-$ | 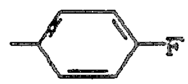 | 190° C |
| 65 | H | $-CH_3$ | H | H | 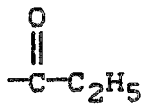 | $-CH_2-$ | 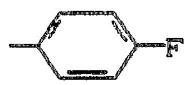 | 132° C |
| 66 | H | $-CH_3$ | H | H | 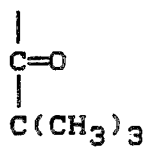 | $-CH_2-$ | 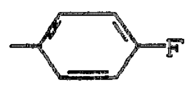 | 90° C |
| 67 | H | $-CH_3$ | H | H | 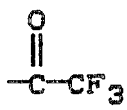 | $-CH_2-$ | 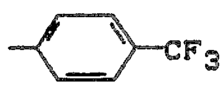 | 107° C |
| 68 | H | $-CH_3$ | H | H | 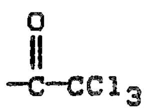 | $-CH_2-$ | 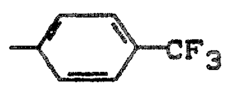 | 143° C |
| 69 | H | $-CH_3$ | H | H | 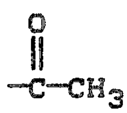 | $-CH_2-$ | 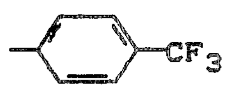 | 135° C |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H-NMR$* |
|---|---|---|---|---|---|---|---|---|
| 70 | H | $-CH_3$ | H | H | 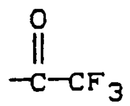 | $-CH_2-$ | 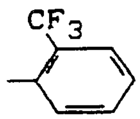 | 149° C |
| 71 | H | $-CH_3$ | H | H | 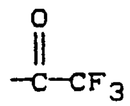 | $-CH_2-$ | 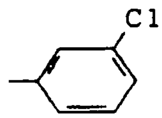 | 131° C |
| 72 | H | $-CH_3$ | H | H | 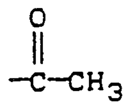 | $-CH_2-$ | 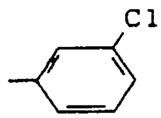 | 88° C |
| 73 | H | $-CH_3$ | H | H | 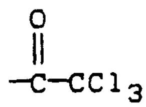 | $-CH_2-$ | 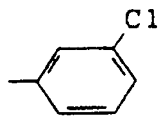 | δ=5,56 |
| 74 | H | H | H | H | 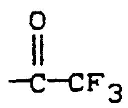 | $-CH_2-$ | 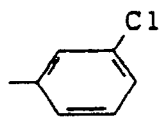 | 140° C |
| 75 | H | H | H | H | 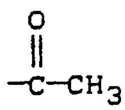 | $-CH_2-$ | 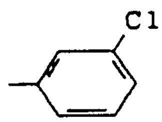 | 92° C |
| 76 | H | H | H | H | 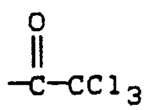 | $-CH_2-$ | 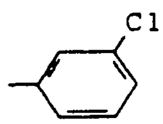 | 140° C |
| 77 | H | Cl | H | H | 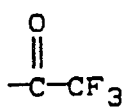 | $-CH_2-$ | 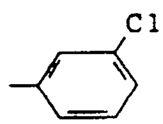 | 152° C |

<u>Tabelle 2</u> (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 78 | H | Cl | H | H | 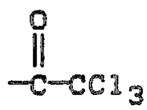 | $-CH_2-$ | 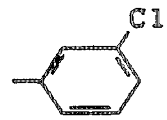 | 185° C |
| 79 | H | $-CH_3$ | H | H | 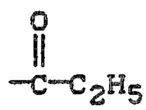 | $-CH_2-$ | 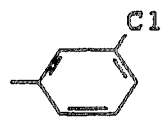 | 114° C |
| 80 | H | $-CH_3$ | H | H | 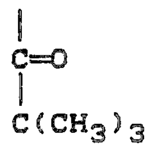 | $-CH_2-$ | 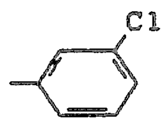 | 141° C |
| 81 | H | Cl | H | H | 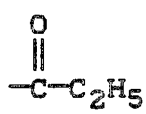 | $-CH_2-$ | 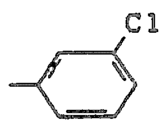 | 111° C |
| 82 | H | Cl | H | H | 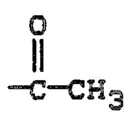 | $-CH_2-$ | 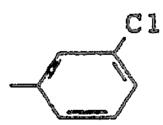 | δ=5,33 |
| 83 | H | $CH_3$ | H | H | 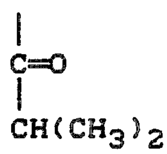 | $-CH_2-$ | 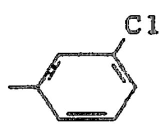 | 110° C |
| 84 | H | $-CH_3$ | H | H | 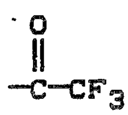 | $-CH_2-$ | 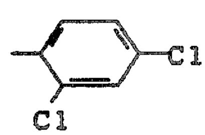 | 125° C |
| 85 | H | $-CH_3$ | H | H | 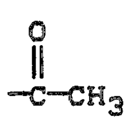 | $-CH_2-$ | 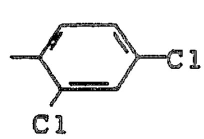 | 136° C |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 86 | H | $-CH_3$ | H | H | $-SO_2CH_3$ | $-CH_2-$ | 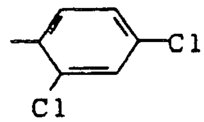 | 176° C |
| 87 | H | $-CH_3$ | H | H | 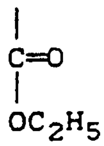 | $-CH_2-$ | 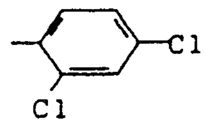 | 165° C |
| 88 | H | $-CH_3$ | H | H | 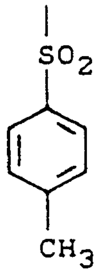 | $-CH_2-$ | 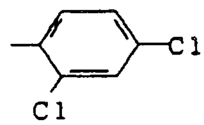 | 168° C |
| 89 | H | $-CH_3$ | H | H | 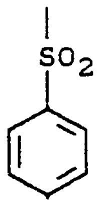 | $-CH_2-$ | 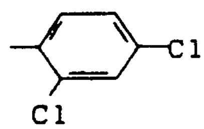 | 169° C |
| 90 | H | $-CH_3$ | H | H | 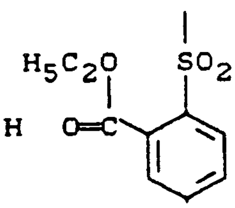 | $-CH_2-$ | 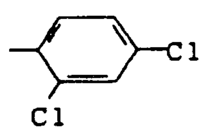 | 152° C |
| 91 | H | Cl | H | H | 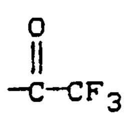 | $-CH_2-$ | 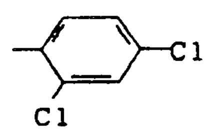 | 124° C |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 92 | H | Cl | H | H | 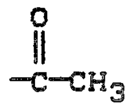 | $-CH_2-$ | 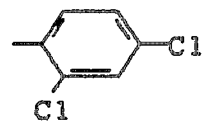 | 135° C |
| 93 | H | H | H | H | 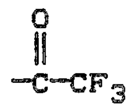 | $-CH_2-$ | 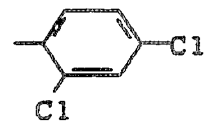 | δ=5,68 (DMSO) |
| 94 | H | $-CH_3$ | H | H | 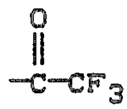 | $-CH_2-$ | 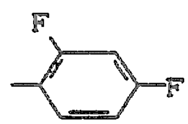 | 149° C |
| 95 | H | $-CH_3$ | H | H | 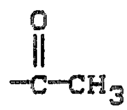 | $-CH_2-$ | 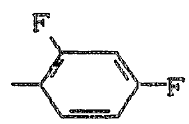 | δ=5,37 |
| 96 | H | $-CH_3$ | H | H | 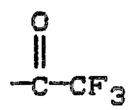 | $-CH_2-$ | 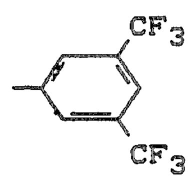 | δ=5,59 |
| 97 | H | $-CH_3$ | H | H | 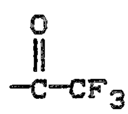 | $-CH_2-$ | 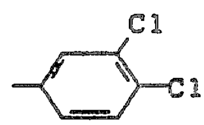 | 160° C |
| 98 | H | $-CH_3$ | H | H | 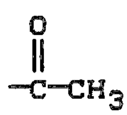 | $-CH_2-$ | 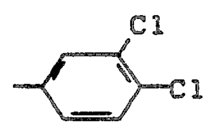 | 142° C |
| 99 | H | $-CH_3$ | H | H | 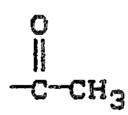 | $-CH_2-$ | 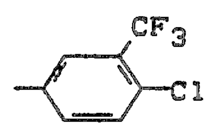 | 140° C |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 100 | H | $-CH_3$ | H | H | 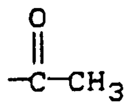 | $-CH_2-$ | 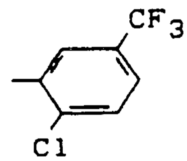 | 150° C |
| 101 | H | $-CH_3$ | H | H | 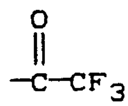 | $-CH_2-$ | 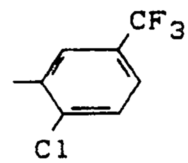 | 138° C |
| 102 | H | $-CH_3$ | H | H | 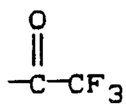 | $-CH_2-$ | 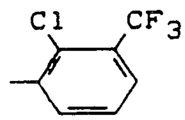 | 154° C |
| 103 | H | $-CH_3$ | H | H | 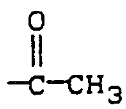 | $-CH_2-$ | 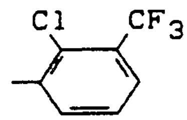 | 147° C |
| 104 | H | $-CH_3$ | H | H | 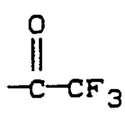 | $-CH_2-$ | 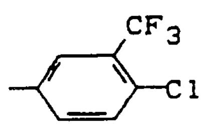 | 104° C |
| 105 | H | $-CH_3$ | H | H | 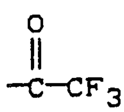 | $-CH_2-$ | 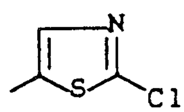 | 198° C |
| 106 | H | $-CH_3$ | H | H | 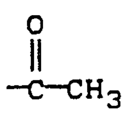 | $-CH_2-$ | 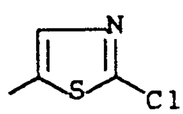 | 183° C |
| 107 | H | $-CH_3$ | H | H | 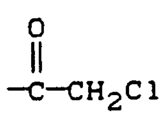 | $-CH_2-$ | 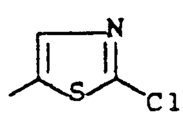 | $\delta$=5,48 |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 108 | H | $-CH_3$ | H | H | 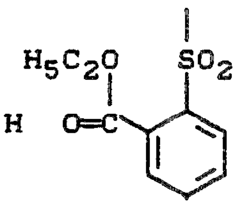 | $-CH_2-$ | 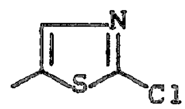 | δ=5,35 |
| 109 | H | $-CH_3$ | H | H | $-CN$ | $-CH_2-$ | 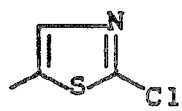 | 205° C |
| 110 | H | H | H | H | 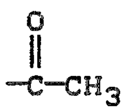 | $-CH_2-$ | 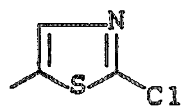 | δ=5,35 |
| 111 | H | H | H | H | 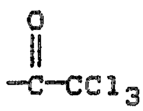 | $-CH_2-$ | 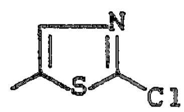 | 192° C |
| 112 | H | H | H | H | 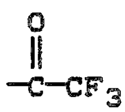 | $-CH_2-$ | 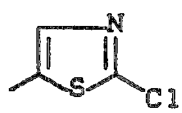 | 146° C |
| 113 | H | Cl | H | H | 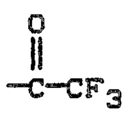 | $-CH_2-$ | 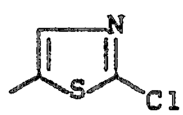 | 170° C |
| 114 | H | Cl | H | H | 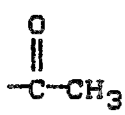 | $-CH_2-$ | 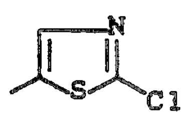 | δ=5,30 |
| 115 | H | Cl | H | H | 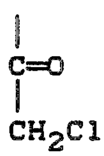 | $-CH_2-$ | 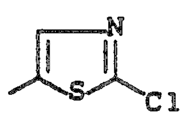 | 176° C |

<u>Tabelle 2</u> (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 116 | H | Cl | H | H | 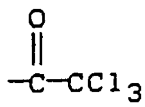 | $-CH_2-$ | 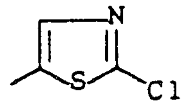 | 169° C |
| 117 | H | Cl | H | H | $-CN$ | $-CH_2-$ | 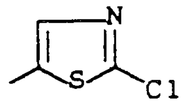 | 214° C |
| 118 | H | H | $-CH_3$ | H | 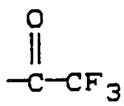 | $-CH_2-$ | 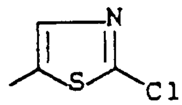 | $\delta$=5,52 |
| 119 | H | H | $-CH_3$ | H | 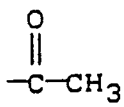 | $-CH_2-$ | 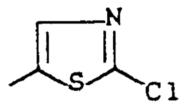 | 238° C |
| 120 | H | H | $-CH_3$ | H | $-SO_2-CH_3$ | $-CH_2-$ | 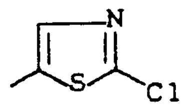 | $\delta$=5,29 |
| 121 | H | H | $-CH_3$ | H | 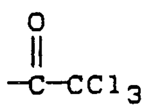 | $-CH_2-$ | 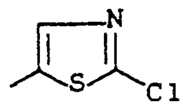 | 163° C |
| 122 | H | H | H | $-CH_3$ | 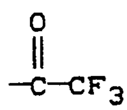 | $-CH_2-$ | 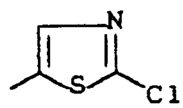 | $\delta$=5,57 |
| 123 | H | H | H | $-CH_3$ | 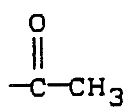 | $-CH_2-$ | 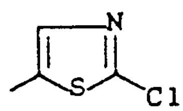 | $\delta$=5,40 |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 124 | H | H | H | $-CH_3$ | $-SO_2-CH_3$ | $-CH_2-$ | 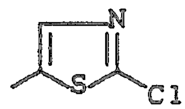 | 160° C |
| 125 | H | $-CH_3$ | H | H | C=O<br>$C_2H_5$ | $-CH_2-$ | 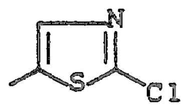 | 197° C |
| 126 | H | H | H | H | C=O<br>$C_2H_5$ | $-CH_2-$ | 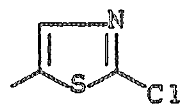 | 183° C |
| 127 | H | $-CH_3$ | H | H | C=O<br>$C(CH_3)_3$ | $-CH_2-$ | 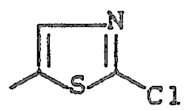 | 120° C |
| 128 | H | $-CH_3$ | H | H | C=O<br>$CH(CH_3)_2$ | $-CH_2-$ | 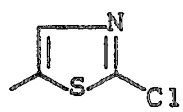 | 118° C |
| 129 | H | Cl | H | H | C=O<br>$C(CH_3)_3$ | $-CH_2-$ | 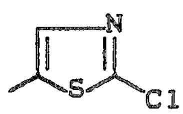 | 107° C |
| 130 | H | Cl | H | H | C=O<br>$CH(CH_3)_2$ | $-CH_2-$ | 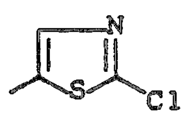 | 114° C |
| 131 | H | Cl | H | H | C=O<br>$OC_2H_5$ | $-CH_2-$ | 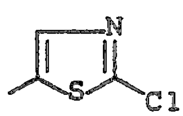 | 149° C |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 132 | H | $-CH_3$ | H | H | 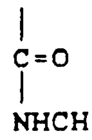 | $-CH_2-$ | 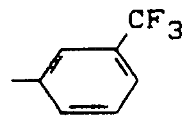 | δ=5,30 |
| 133 | H | $-CH_3$ | H | H | 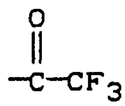 | $-CH_2-$ | 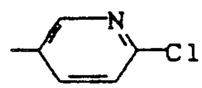 | 140° C |
| 134 | H | H | H | H | 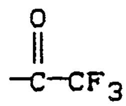 | $-CH_2-$ | 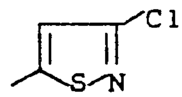 | 153° C |
| 135 | H | $-CH_3$ | H | H | 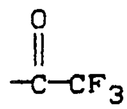 | $-CH_2-$ | 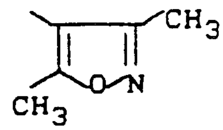 | 128° C |
| 136 | H | H | $-CH_3$ | H | 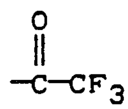 | $-CH_2-$ | 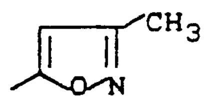 | 113° C |
| 137 | H | $-CH_3$ | H | H | 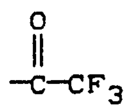 | $-CH_2-$ | 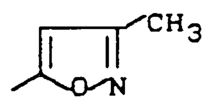 | 137° C |
| 138 | H | H | H | H | 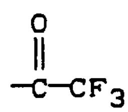 | $-CH_2-$ | 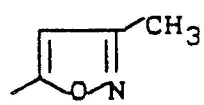 | 171° C |
| 139 | H | $-CH_3$ | H | H | 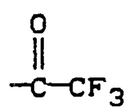 | $-CH_2-$ | 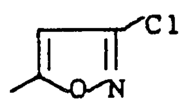 | 146° C |
| 140 | H | H | $-CH_3$ | H | 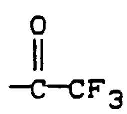 | $-CH_2-$ | 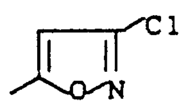 | 143° C |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 141 | H | H | H | H | 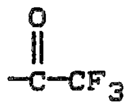 | $-CH_2-$ | 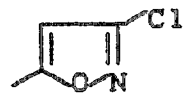 | 170° C |
| 142 | H | H | H | H | 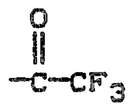 | $-CH_2-$ | 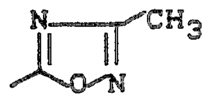 | 135° C |
| 143 | H | H | $-CH_3$ | H | 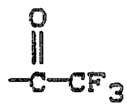 | $-CH_2-$ | 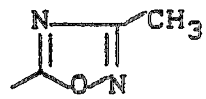 | 127° C |
| 144 | H | $-CH_3$ | H | H | 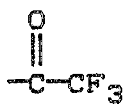 | $-CH_2-$ | 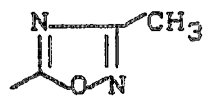 | 154° C |
| 145 | H | Cl | H | H | 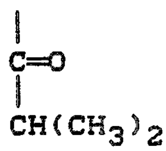 | $-CH_2-$ | 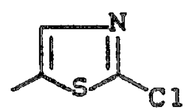 | 120° C |
| 146 | H | $-CH_3$ | H | H | 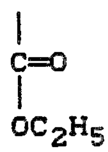 | $-CH_2-$ | 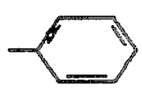 | 109° C |
| 147 | H | $-CH_3$ | H | H | 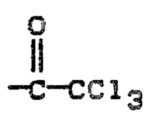 | $-CH_2-$ | 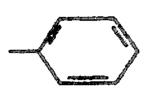 | 138° C |
| 148 | H | $-CH_3$ | H | H | 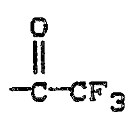 | $-CH_2-$ | 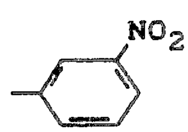 | 158° C |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$–NMR* |
|---|---|---|---|---|---|---|---|---|
| 149 | H | $-CH_3$ | H | H | 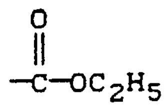 | $-CH_2-$ | 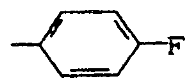 | 104° C |
| 150 | H | $-CH_3$ | H | H | 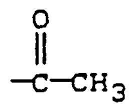 | $-CH_2-$ | 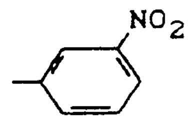 | 155° C |
| 151 | H | $-CH_3$ | H | H | 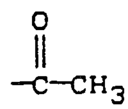 | $-CH_2-$ | 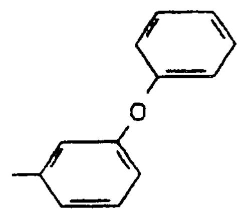 | 150° C |
| 152 | H | $-CH_3$ | H | H | 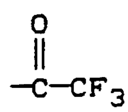 | $-CH_2-$ | 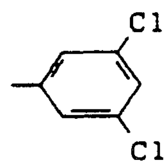 | 186° C |
| 153 | H | $-CH_3$ | H | H | 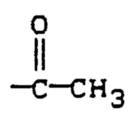 | $-CH_2-$ | 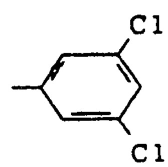 | 173° C |
| 154 | H | $-CH_3$ | H | H | 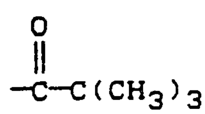 | $-CH_2-$ | 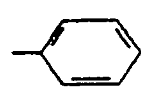 | 80° C |
| 155 | H | $-CH_3$ | H | H | 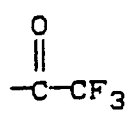 | $-CH_2-$ | 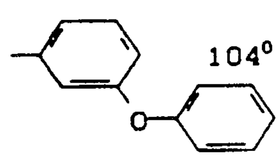 | 104° C |

Tabelle 2 (Fortsetzung)

| Bsp. Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 156 | H | $CH_3$ | H | H | 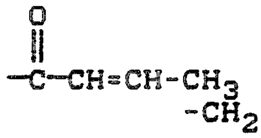 | $-CH_2-$ | 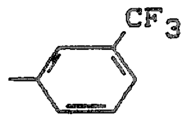 | 124° C |
| 157 | H | $CF_3$ | H | H | 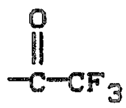 | $-CH_2-$ | 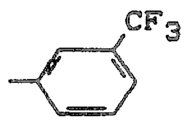 | 108° C |
| 158 | H | $CH_3$ | H | H | 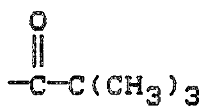 | $-CH_2-$ | 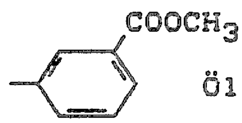 | Öl |
| 159 | H | $CH_3$ | H | H | 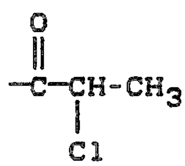 | $-CH_2-$ | 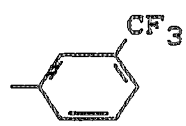 | 80° C |
| 160 | H | $CH_3$ | H | H | $-SO_2-CH_3$ | $-CH_2-$ | 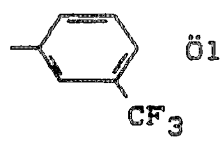 | Öl |
| 161 | H | $CH_3$ | H | H | $-CO-CHCl_2$ | $-CH_2-$ | 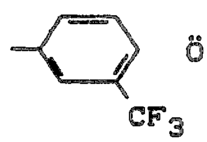 | Öl |
| 162 | H | $CH_3$ | H | H | $-CO-CF_3$ | $-CH_2-$ | 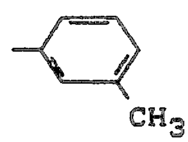 | 92° C |

**Tabelle 2** - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 163 | H | $CH_3$ | H | H | $-CO-CH(CH_3)_2$ | $-CH_2-$ | 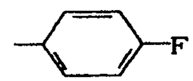 | 66° C |
| 164 | H | Cl | H | H | $-CO-CH(CH_3)_2$ | $-CH_2$ | 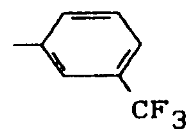 | 83° C |
| 165 | H | $CH_3$ | H | H | -CO 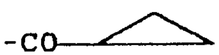 | $-CH_2$ | 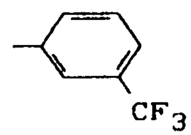 | 76° C |
| 166 | H | $CH_3$ | H | H | -CO 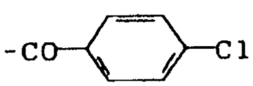 | $-CH_2-$ | 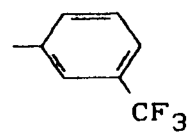 | 181° C |
| 167 | H | $CH_3$ | H | H | -CO 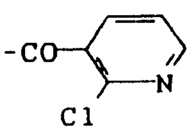 | $-CH_2-$ | 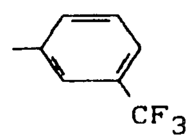 | 127° C |
| 168 | H | $CH_3$ | H | H | $-CO-CF_3$ | $-CH_2-$ | 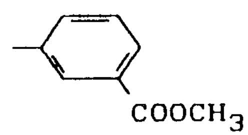 | 102° C |

**Tabelle 2** - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1$H-NMR* |
|---|---|---|---|---|---|---|---|---|
| 169 | H | $CH_3$ | H | H | $-CO-CH_2C_6H_5$ | $-CH_2-$ | 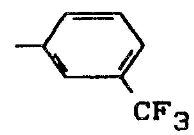 | amorph |
| 170 | H | $CH_3$ | H | H | $-COOC(CH_3)_2C_2H_5$ | $-CH_2-$ | 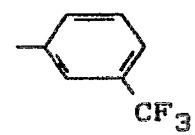 | 105° C |
| 171 | H | $CH_3$ | H | H | $-CO-C_{11}H_{23}$ | $-CH_2-$ | 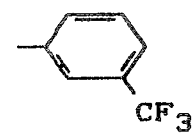 | 52° C |
| 172 | H | $CH_3$ | H | H | $-COOC(CH_3)_3$ | $-CH_2-$ | 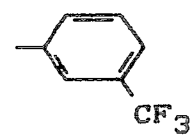 | 143° C |
| 173 | H | $CH_3$ | H | H | $-COOCH_3$ | $-CH_2-$ | 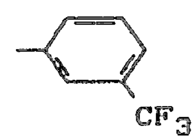 | 94° C |
| 174 | H | $CH_3$ | H | H | $-CO-C_5H_{11}$ | $-CH_2-$ | 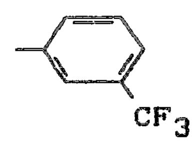 | 56° C |

Tabelle 2 - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 175 | H | $CH_3$ | H | H | $-CO-C_6H_{13}$ | $-CH_2-$ | 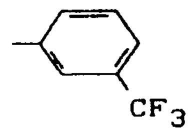 | 74° C |
| 176 | H | $CH_3$ | H | H | 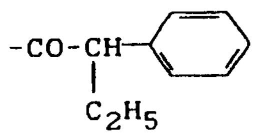 | $-CH_2-$ | 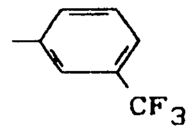 | 135° C |
| 177 | H | $CH_3$ | H | H | $-CO-COOC_2H_5$ | $-CH_2-$ | 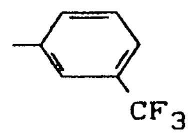 | 153° C |
| 178 | H | $CH_3$ | H | H | $-CO-CH_2CH(CH_3)_2$ | $-CH_2-$ | 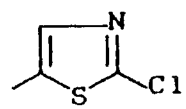 | 112° C |
| 179 | H | $CH_3$ | H | H | 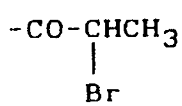 | $-CH_2-$ | 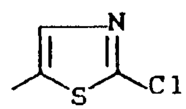 | 122° C |
| 180 | H | $CH_3$ | H | H | $-CO-C_3H_7$ | $-CH_2-$ | 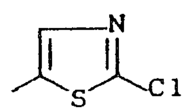 | 138° C |

<u>Tabelle 2</u> - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 181 | H | $CH_3$ | H | H | $-CO-C_4H_9$ | $-CH_2-$ | 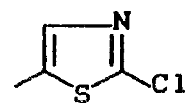 | 133° C |
| 182 | H | $CH_3$ | H | H | $-CO-CH_2C(CH_3)_3$ | $-CH_2-$ | 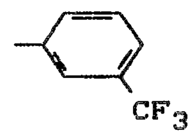 | 96° C |
| 183 | H | $CH_3$ | H | H | $-CO-C(CH_3)_3$ | $-CH_2-$ | 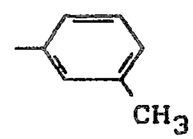 | 121° C |
| 184 | H | $CH_3$ | H | H | $-CO-CH=CHCH_3$ | $-CH_2-$ | 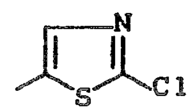 | 180° C |
| 185 | H | $CH_3$ | H | H | -CO 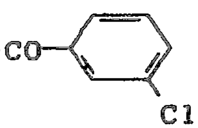 | $-CH_2-$ | 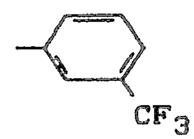 | 115° C |
| 186 | H | $CH_3$ | H | H | -CO 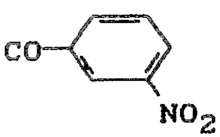 | $-CH_2-$ | 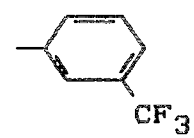 | 149° C |

**Tabelle 2** - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 187 | H | $CH_3$ | H | H | $-CO-C_2F_5$ | $-CH_2-$ | 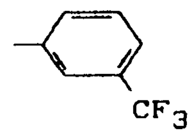 | 70° C |
| 188 | H | $CH_3$ | H | H | 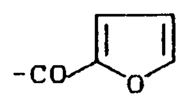 | $-CH_2-$ | 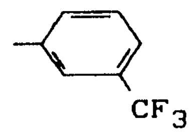 | 144° C |
| 189 | H | $CH_3$ | H | H | 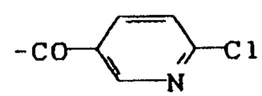 | $-CH_2-$ | 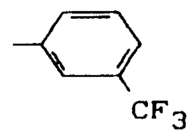 | 157° C |
| 190 | H | $CH_3$ | H | H | 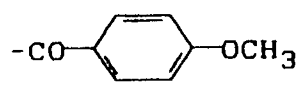 | $-CH_2-$ | 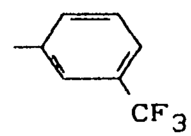 | 123° C |
| 191 | H | $CH_3$ | H | H | 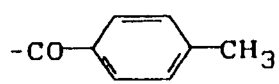 | $-CH_2-$ | 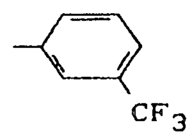 | 138° C |
| 192 | H | $CH_3$ | H | H | 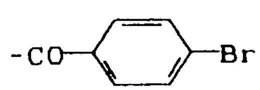 | $-CH_2-$ | 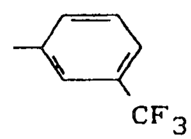 | 168° C |

**Tabelle 2** - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1$H-NMR* |
|---|---|---|---|---|---|---|---|---|
| 193 | H | $CH_3$ | H | H | $-CO-CH_2OCH_3$ | $-CH_2-$ | 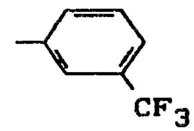 | 140° C |
| 194 | H | $CH_3$ | H | H | -CO 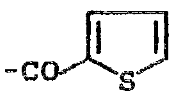 | $-CH_2-$ | 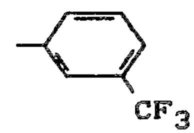 | 130° C |
| 195 | H | $CH_3$ | H | H | $-CO-CH=CHCH_3$ | $-CH_2-$ | $C_6H_5$ | 57° C |
| 196 | H | $CH_3$ | H | H | -CO 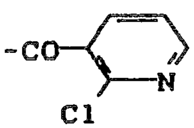 | $-CH_2-$ | $C_6H_5$ | amorph |
| 197 | H | $CH_3$ | H | H | $-COOCH_3$ | $-CH_2-$ | $C_6H_5$ | 72° C |
| 198 | H | $CH_3$ | H | H | $-CO-CH=CHCH_3$ | $-CH_2-$ | 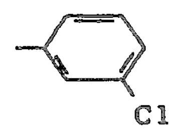 | 105° C |
| 199 | H | $CH_3$ | H | H | $-COOCH_3$ | $-CH_2-$ | 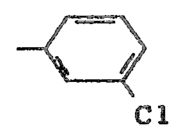 | 95° C |

Tabelle 2 - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 200 | H | $CH_3$ | H | H | -CO 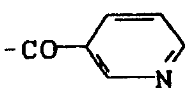 | $-CH_2-$ | 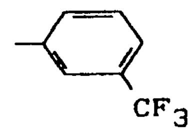 | 139° C |
| 201 | H | $CH_3$ | H | H | -CO 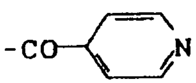 | $-CH_2-$ | 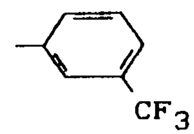 | 190° C |
| 202 | H | $CH_3$ | H | H | $-CO-CF_3$ | $-CH_2-$ | 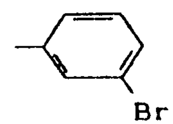 | 122° C |
| 203 | H | $CH_3$ | H | H | $-COOC_2H_5$ | $-CH_2-$ | 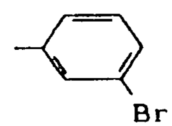 | 59° C |
| 204 | H | $CH_3$ | H | H | $-CO-C(CH_3)_3$ | $-CH_2-$ | 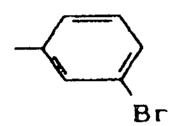 | 113° C |
| 205 | H | $CH_3$ | H | H | -CO 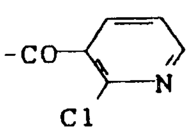 | $-CH_2-$ | 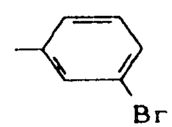 | 155° C |

Tabelle 2 - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1$H-NMR* |
|---|---|---|---|---|---|---|---|---|
| 206 | H | $CH_3$ | H | H | $-CO-CF_3$ | $-CH_2-$ | 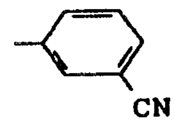 | 129° C |
| 207 | H | $CH_3$ | H | H | $-CO-C(CH_3)_3$ | $-CH_2-$ | 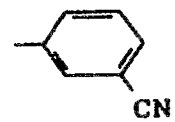 | 96° C |
| 208 | H | $CH_3$ | H | H | $-COOC_2H_5$ | $-CH_2-$ | 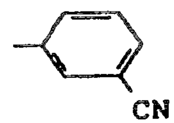 | 116° C |
| 209 | H | $CH_3$ | H | H | -CO 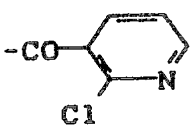 | $-CH_2-$ | 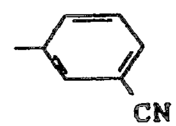 | 144° C |
| 210 | H | $CH_3$ | H | H | $-CO-C_2H_5$ | $-CH_2-$ | 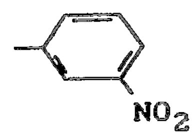 | 128° C |
| 211 | H | $CH_3$ | H | H | $-CO-C_3H_7$ | $-CH_2-$ | 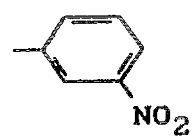 | 104° C |

**Tabelle 2 - Fortsetzung**

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 212 | H | $CH_3$ | H | H | $-CO-CH(CH_3)_2$ | $-CH_2-$ | 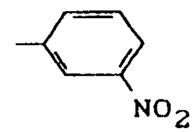 | 80° C |
| 213 | H | $CH_3$ | H | H | $-CO-CH=CHCH_3$ | $-CH_2-$ | 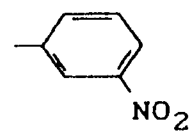 | 132° C |
| 214 | H | $CH_3$ | H | H | $-CO-C_4H_9$ | $-CH_2-$ | 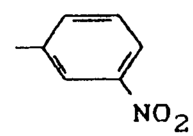 | 94° C |
| 215 | H | $CH_3$ | H | H | $-CO-CH_2CH(CH_3)_2$ | $-CH_2-$ | 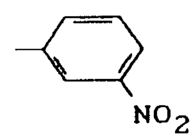 | 96° C |
| 216 | H | $CH_3$ | H | H | $-CO-C(CH_3)_3$ | $-CH_2-$ | 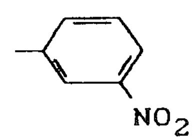 | 98° C |
| 217 | H | $CH_3$ | H | H | $-COOCH_3$ | $-CH_2-$ | 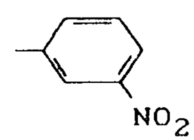 | 141° C |

<u>Tabelle 2</u> - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1$H-NMR* |
|---|---|---|---|---|---|---|---|---|
| 218 | H | $CH_3$ | H | H | $-COOC_2H_5$ | $-CH_2-$ | 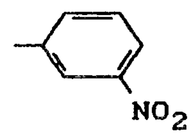 | 131° C |
| 219 | H | $CH_3$ | H | H | $-CO-C_6H_5$ | $-CH_2-$ | 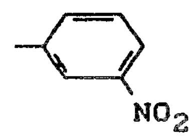 | 137° C |
| 220 | H | $CH_3$ | H | H | $-COOC_2H_5$ | $-CH_2-$ | 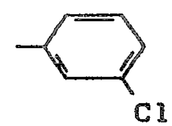 | 71° C |
| 221 | H | $CH_3$ | H | H | $-CO-CH=CHC_6H_5$ | $-CH_2-$ | 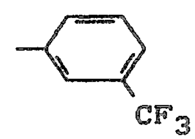 | 118° C |
| 222 | H | Cl | H | H | $-CO-CF_3$ | $-CH_2-$ | 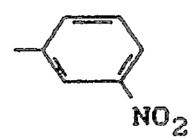 | 136° C |
| 223 | H | $CH_3$ | H | H | $-CO-CF_3$ | $-CH_2-$ | 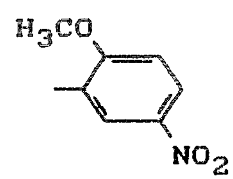 | 196° C |

Tabelle 2 - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 224 | H | $CH_3$ | H | H | $-CO-C(CH_3)_3$ | $-CH_2-$ | 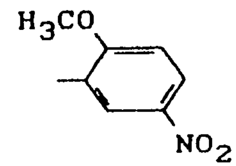 | 189° C |
| 225 | H | $CH_3$ | H | H | $-CO-CF_3$ | $-CH_2-$ | 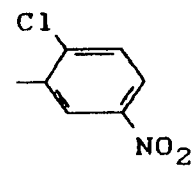 | 140° C |
| 226 | H | $CH_3$ | H | H | $-CO-C(CH_3)_3$ | $-CH_2-$ | 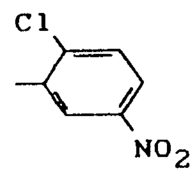 | 162° C |
| 227 | H | $CH_3$ | H | H | $-CO-CH_2OCH_3$ | $-CH_2-$ | 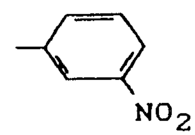 | 150° C |
| 228 | H | $CH_3$ | H | H | $-CO-CH_2OC_6H_5$ | $-CH_2-$ | 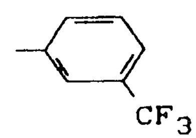 | 113° C |

**Tabelle 2** - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1$H-NMR* |
|---|---|---|---|---|---|---|---|---|
| 229 | H | $CH_3$ | H | H | $-CO-CH_2O$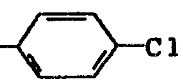 | $-CH_2-$ | 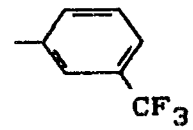 | 96° C |
| 230 | H | $CH_3$ | H | H | $-CO-CH_2OCOCH_3$ | $-CH_2-$ | 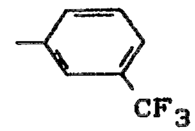 | 84° C |
| 231 | H | $CH_3$ | H | H | 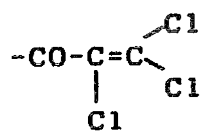 | $-CH_2-$ | 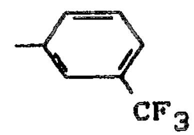 | 102° C |
| 232 | H | $CH_3$ | H | H | 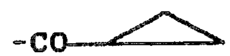 | $-CH_2-$ | 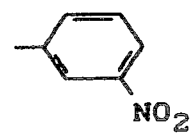 | 112° C |
| 233 | H | $CH_3$ | H | H | 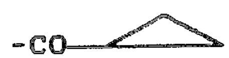 | $-CH_2-$ | 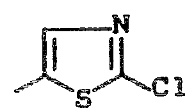 | 150° C |
| 234 | H | $CH_3$ | H | H | 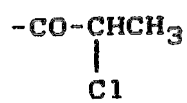 | $-CH_2-$ | 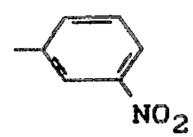 | 111° C |

**Tabelle 2** - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 235 | H | $CH_3$ | H | H | $-CO-\underset{Cl}{CH}CH_3$ | $-CH_2-$ | 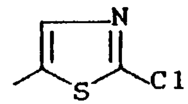 | 109° C |
| 236 | H | $CF_3$ | H | H | $-CO-CF_3$ | $-CH_2-$ | 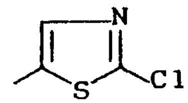 | 112° C |
| 237 | H | $CF_3$ | H | H | $-CO-C(CH_3)_3$ | $-CH_2-$ | 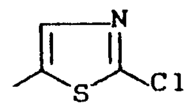 | 114° C |
| 238 | H | $CF_3$ | H | H | -CO 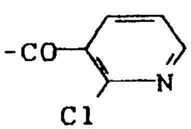 | $-CH_2-$ | 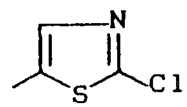 | 86° C |
| 239 | H | $CH_3$ | H | H | -CO 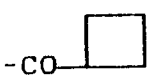 | $-CH_2-$ | 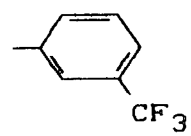 | 107° C |
| 240 | H | $CF_3$ | H | H | $-COOC_2H_5$ | $-CH_2-$ | 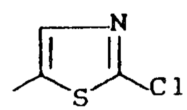 | 127° C |

<u>Tabelle 2</u> - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 241 | H | $CH_3$ | H | H | $-CO-CF_3$ | $-CH_2-$ | 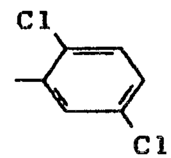 | 98° C |
| 242 | H | $CH_3$ | H | H | $-CO-C(CH_3)_2C_2H_5$ | $-CH_2-$ | 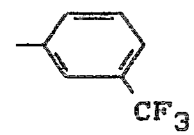 | 46° C |
| 243 | H | $CH_3$ | H | H | $-CO-CH=CH_2$ | $-CH_2-$ | 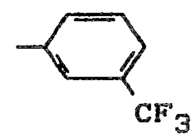 | 68° C |
| 244 | H | $CH_3$ | H | H | $-CO-CHOC_6H_5$<br>$CH_3$ | $-CH_2-$ | 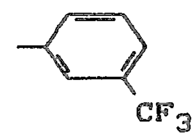 | 103° C |
| 245 | H | $CH_3$ | H | H | $-CO-CF_3$ | $-CH_2-$ | 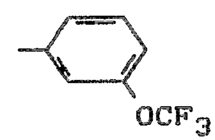 | 98° C |

Tabelle 2 - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 246 | H | $CH_3$ | H | H | $-CO-C(CH_3)_3$ | $-CH_2-$ | 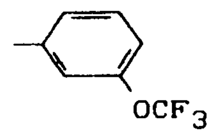 | 101° C |
| 247 | H | Cl | H | H | $-COOC_2H_5$ | $-CH_2-$ | 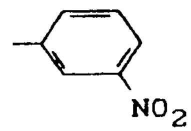 | 128° C |
| 248 | H | Cl | H | H | $-CO-CF_3$ | $-CH_2-$ | 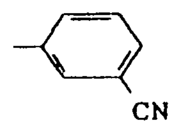 | 137° C |
| 249 | H | Cl | H | H | $-CO-C(CH_3)_3$ | $-CH_2-$ | 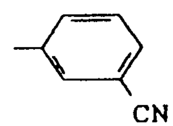 | 126° C |
| 250 | H | Cl | H | H | $-COOC_2H_5$ | $-CH_2-$ | 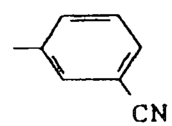 | 102° C |

Tabelle 2 - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1$H-NMR* |
|---|---|---|---|---|---|---|---|---|
| 251 | H | $CH_3$ | H | H | -CO 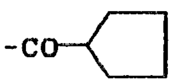 | $-CH_2$ | 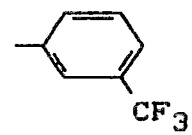 | 95° C |
| 252 | H | $CH_3$ | H | H | $-CO-CF_3$ | $-CH_2-$ | 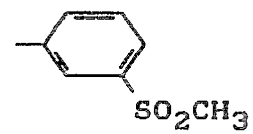 | 105° C |
| 253 | H | $CH_3$ | H | H | -CO 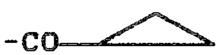 | $-CH_2$ | 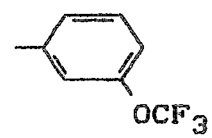 | 72° C |
| 254 | H | H | H | H | -CO 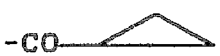 | $-CH_2$ | 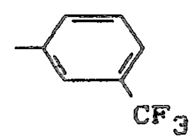 | 101° C |
| 255 | H | $CH_3$ | H | H | -CO 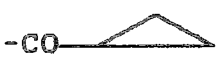 | $-CH_2$ | 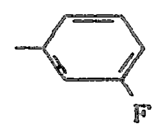 | 73° C |

<u>Tabelle 2</u> - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1$H-NMR* |
|---|---|---|---|---|---|---|---|---|
| 256 | H | Cl | H | H | -CO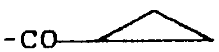 | $-CH_2$ | 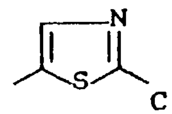 | 148° C |
| 257 | H | $CH_3$ | H | H | -CO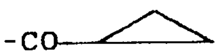 | $-CH_2$ | 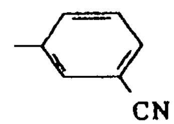 | 74° C |
| 258 | H | H | $CH_3$ | H | -CO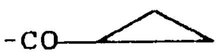 | $-CH_2$ | 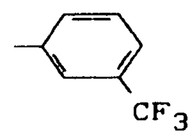 | |
| 259 | H | $CH_3$ | H | H | -CO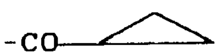 | $-CH_2$ | 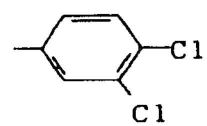 | |

<u>Tabelle 2</u> - Fortsetzung

| Bsp.-Nr. | $R^1$ | $R^2$ | $R^3$ | $R^4$ | $R^5$ bzw. $R^{5-2}$ | A | Z | Schmelzpunkt bzw. $^1H$-NMR* |
|---|---|---|---|---|---|---|---|---|
| 260 | H | $CH_3$ | H | H | -CO 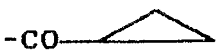 | $-CH_2$ | 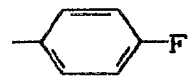 | |
| 261 | H | H | H | H | -CO 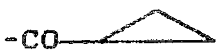 | $-CH_2$ | 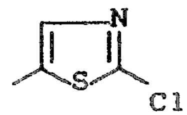 | |
| 262 | H | $CH_3$ | H | H | -CO 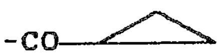 | $-CH_2$ | 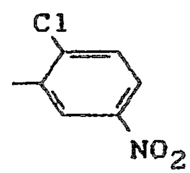 | 173° C |
| 263 | H | $CF_3$ | H | H | -CO 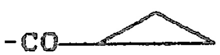 | $-CH_2$ | 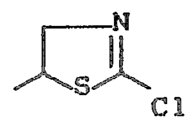 | 124° C |

*) Die $^1H$-NMR-Spektren wurden, sofern nichts anderes angegeben ist, in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen

Beispiel II-I

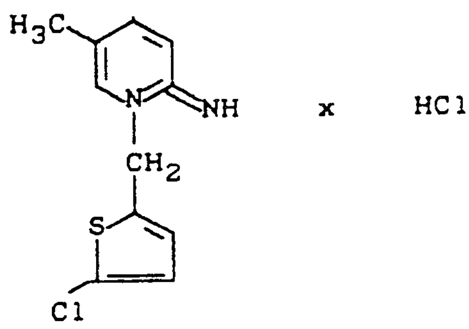

3,2 g (0,03 Mol) 2-Amino-5-methylpyridin werden in 100 ml Acetonitril gelöst und mit 5 g (0,03 Mol) 2-Chlor-5-chlormethylthiophen versetzt. Die Mischung wird 8 Stunden bei 60° C und 18 Stunden bei Raumtemperatur gerührt. Der gebildete Feststoff wird abgesaugt und getrocknet.

Man erhält 1,6 g (20 % der Theorie) an N-(5-Chlorthien-2-yl-methyl)-5-methyl-2-iminopyridin-hydrochlorid vom Schmelzpunkt 208° C.

In entsprechender Weise und gemäß den allgemeinen Angaben zur Herstellung erhält man die in Tabelle 3 aufgeführten Verbindungen der Formel (II).

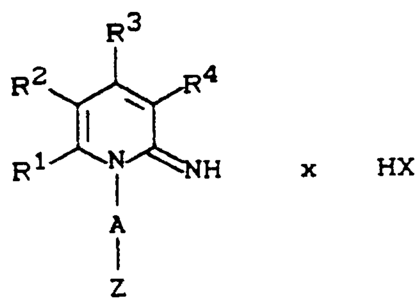

Tabelle 3

| Bsp. Nr. | $R^1$ | $R^2$ | $R^3$ | $R^4$ | A | Z | HX | Schmelzpunkt bzw. $^1$H–NMR* |
|---|---|---|---|---|---|---|---|---|
| II-2 | H | $CH_3$ | H | H | $-CH_2-$ | 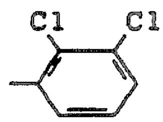 | HCl | 248° C |
| II-3 | H | H | H | H | $-CH_2-$ | 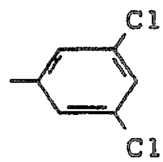 | HCl | Zers 218° C |
| II-4 | H | H | H | H | $-CH_2-$ | 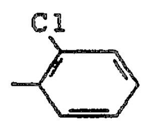 | HCl | δ=5,59 |
| II-5 | H | $CH_3$ | H | H | $-CH_2-$ | 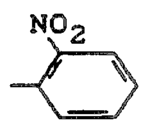 | HCl | 250° C |
| II-6 | H | $CH_3$ | H | H | $-CH_2-$ | 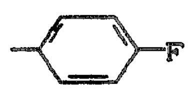 | HCl | 172° C |
| II-7 | H | $CH_3$ | H | H | $-CH_2-$ | 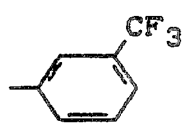 | HCl | 195 - 197° C |
| II-8 | H | Cl | H | H | $-CH_2-$ | 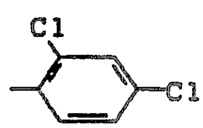 | HCl | δ=5,51 |
| II-9 | H | H | H | H | $-CH_2-$ | 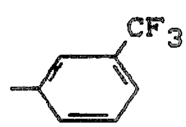 | HCl | 180° C |

Tabelle 3 (Fortsetzung)

| Bsp. Nr. | $R^1$ | $R^2$ | $R^3$ | $R^4$ | A | Z | HX | Schmelzpunkt bzw. $^1$H-NMR* |
|---|---|---|---|---|---|---|---|---|
| II-10 | H | Cl | H | H | $-CH_2-$ | 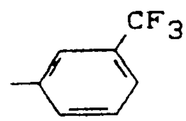 | HCl | δ=5,65 |
| II-11 | H | $CH_3$ | H | H | $-CH_2-$ | 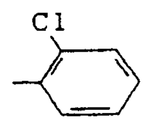 | HCl | δ=5,57 |
| II-12 | H | $CH_3$ | H | H | $-CH_2-$ | 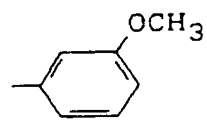 | HCl | 155° C |
| II-13 | H | $CH_3$ | H | H | $-CH_2-$ | 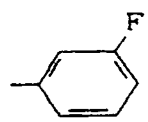 | HCl | δ=5,59 |
| II-14 | H | $CH_3$ | H | H | $-CH_2-$ | 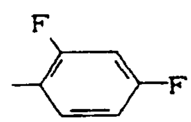 | HCl | 239° C |
| II-15 | H | $CH_3$ | H | H | $-CH_2-$ | 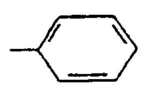 | HBr | 216° C |
| II-16 | H | H | $CH_3$ | H | $-CH_2-$ | 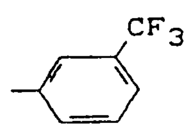 | HCl | δ=5,61 |
| II-17 | H | $CH_3$ | H | H | $-CH_2-$ | 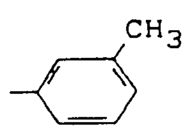 | HCl | δ=5,42 |

Tabelle 3 (Fortsetzung)

| Bsp. Nr. | $R^1$ | $R^2$ | $R^3$ | $R^4$ | A | Z | HX | Schmelzpunkt bzw. $^1H$-NMR* |
|---|---|---|---|---|---|---|---|---|
| II-18 | H | H | H | H | $-CH_2-$ | 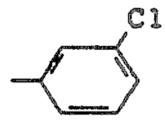 | HCl | δ=5,55 |
| II-19 | H | H | H | H | $-CH_2-$ | 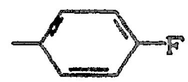 | HCl | δ=5,59 |
| II-20 | H | $CH_3$ | H | H | $-CH_2-$ | 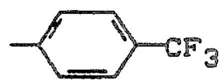 | HCl | 240° C |
| II-21 | H | $CH_3$ | H | H | $-CH_2-$ | 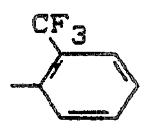 | HCl | 230° C |
| II-22 | H | $CH_3$ | H | H | $-CH_2-$ | 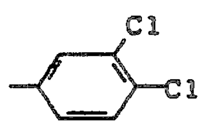 | HCl | δ=5,52 |
| II-23 | H | $CH_3$ | H | H | $-CH_2-$ | 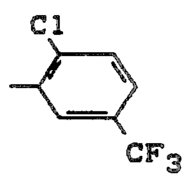 | HCl | δ=5,59 |
| II-24 | H | $CH_3$ | H | H | $-CH_2-$ | 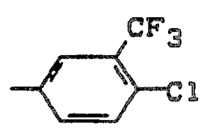 | HCl | δ=5,62 |

Tabelle 3 (Fortsetzung)

| Bsp. Nr. | $R^1$ | $R^2$ | $R^3$ | $R^4$ | A | Z | HX | Schmelzpunkt bzw. $^1H-NMR$* |
|---|---|---|---|---|---|---|---|---|
| II-25 | H | $CH_3$ | H | H | $-CH_2-$ | 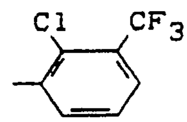 | HCl | δ=5,59 |
| II-26 | H | H | H | H | $-CH_2-$ | 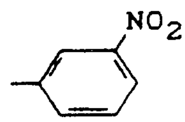 | HCl | 212° C |
| II-27 | H | $CH_3$ | H | H | $-CH_2-$ | 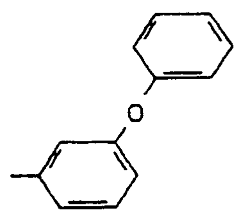 | HCl | 210° C |
| II-28 | H | $CH_3$ | H | H | $-CH_2-$ | 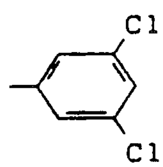 | HCl | 280° C |
| II-29 | H | $CH_3$ | H | H | $-CH_2-$ | 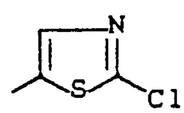 | HCl | δ=5,82 |
| II-30 | H | H | $CH_3$ | H | $-CH_2-$ | 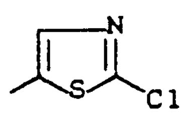 | HCl | 112° C |
| II-31 | H | H | H | $-CH_3$ | $-CH_2-$ | 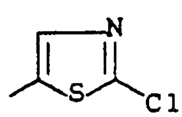 | HCl | 204° C |

Tabelle 3 (Fortsetzung)

| Bsp. Nr. | $R^1$ | $R^2$ | $R^3$ | $R^4$ | A | Z | HX | Schmelzpunkt bzw. $^1H$-NMR* |
|---|---|---|---|---|---|---|---|---|
| II-32 | H | Cl | H | H | $-CH_2-$ | 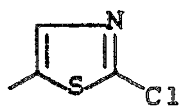 | HCl | 230° C |
| II-33 | H | H | H | H | $-CH_2-$ | 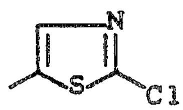 | HCl | δ=5,86 |
| II-34 | H | $CH_3$ | H | H | $-CH_2-$ | 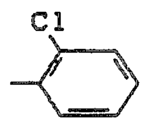 | HCl | δ=5,50 |
| II-35 | H | Cl | H | H | $-CH_2-$ | 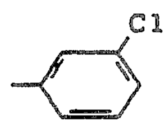 | HCl | δ=5,59 |
| II-36 | H | Cl | H | H | $-CH_2-$ | 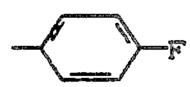 | HCl | δ=5,45 |
| II-37 | H | $CH_3$ | H | H | $-CH_2-$ | 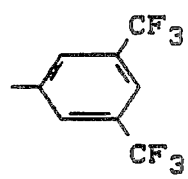 | HCl | δ=5,77 |
| II-38 | H | $CH_3$ | H | H | $-CH_2-$ | 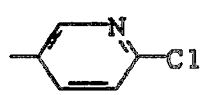 | HCl | Öl |
| II-39 | H | H | H | H | $-CH_2-$ | 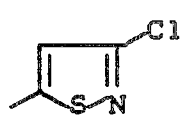 | HBr | Öl |

Tabelle 3 (Fortsetzung)

| Bsp. Nr. | $R^1$ | $R^2$ | $R^3$ | $R^4$ | A | Z | HX | Schmelzpunkt bzw. $^1H$-NMR* |
|---|---|---|---|---|---|---|---|---|
| II-40 | H | $CH_3$ | H | H | $-CH_2-$ | 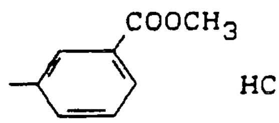 | HCl | 200° C |
| II-41 | H | Cl | H | H | $-CH_2-$ | 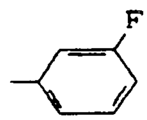 | HCl | 168° C |
| II-42 | H | $CF_3$ | H | H | $-CH_2-$ | 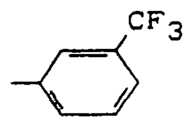 | HCl | 265° C |
| II-43 | H | Cl | H | H | $-CH_2-$ | 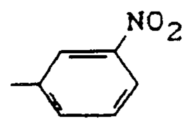 | HCl | 261° C |
| II-44 | H | $CH_3$ | H | H | $-CH_2-$ | 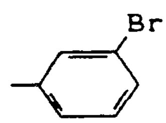 | HBr | 218° C |
| II-45 | H | $CH_3$ | H | H | $-CH_2-$ | 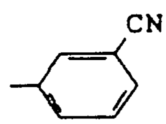 | HCl | 233° C |
| II-46 | H | $CH_3$ | H | H | $-CH_2-$ | 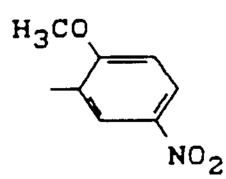 | HCl | 222° C |
| II-47 | H | $CF_3$ | H | H | $-CH_2-$ | 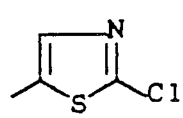 | HCl | 201° C |

Tabelle 3 (Fortsetzung)

| Bsp. Nr. | $R^1$ | $R^2$ | $R^3$ | $R^4$ | A | Z | HX | Schmelzpunkt bzw. $^1H$-NMR* |
|---|---|---|---|---|---|---|---|---|
| II-48 | H | $CH_3$ | H | H | $-CH_2-$ | 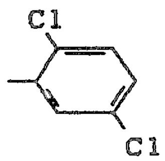 | HCl | 225° C |
| II-49 | H | $CH_3$ | H | H | $-CH_2-$ | 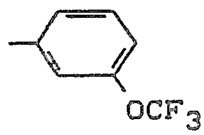 | HCl | 106° C |

*) Die $^1H$-NMR-Spektren wurden in Dimethylsulfoxid (DMSO) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurde die nachstehend aufgeführte Verbindung als Vergleichsubstanz eingesetzt:

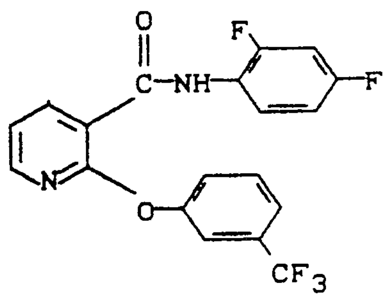

(A)

N-(2,4-Difluorphenyl)-2-[3-(trifluormethyl)-phenoxy]-3-pyridincarboxamid (bekannt aus EP-A 53 011/Verbindung Nr. 3).

Beispiel A

Post-emergence-Test

**Lösungsmittel:** 5 Gewichtsteile Aceton

**Emulgator:** 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Wirksamkeit gegenüber der Vergleichssubstanz (A) zeigt in diesem Test z.B. die Verbindung des Herstellungsbeispiels 15.

Auch die Verbindungen gemäß den Herstellungsbeispielen 13, 26, 33, 71, 77, 113, 157, 187 und 202 zeigen in diesem Test bei guter Verträglichkeit gegenüber Kulturpflanzen, wie z.B. Weizen, Gerste und teilweise auch Mais, starke Wirkung gegen Unkräuter.

Beispiel B

Pre-emergence-Test

**Lösungsmittel:** 5 Gewichtsteile Aceton

**Emulgator:** 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Wirksamkeit gegenüber der Vergleichssubstanz (A) zeigen in diesem Test z.B. die Verbindungen gemäß folgender Herstellungsbeispiele: 15 und 105.

Auch die Verbindungen gemäß den Herstellungsbeispielen 1, 2, 3, 13, 26, 28, 33, 43, 47, 50, 77, 125, 128, 130, 131, 139, 148, 157, 164, 165, 167, 173, 178, 180, 181, 184, 187, 188, 190, 193, 202 und 205 zeigen in diesem Test bei teilweise guter Kulturpflanzen-Verträglichkeit starke Wirkung gegen Unkräuter.

## Ansprüche

1. Herbizide Mittel, gekennzeichnet durch einen Gehalt an mindestens einem 2-Iminopyridin-Derivat der Formel (IA)

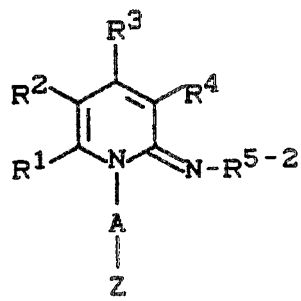

(IA)

in welcher

$R^1$, $R^2$, $R^3$ und $R^4$ unabhängig voneinander für Wasserstoff, Halogen, Alkyl, Halogenalkyl, Alkoxy, Halogenalkoxy, Alkylthio oder Halogenalkylthio stehen,

$R^{5-2}$ für Cyano, Alkylcarbonyl, Cycloalkylcarbonyl, Alkenylcarbonyl, Alkinylcarbonyl, Halogenalkylcarbonyl, Alkoxyalkylcarbonyl, Halogenalkenylcarbonyl, Halogenalkinylcarbonyl, Alkoxycarbonyl, Alkylthiocarbonyl, für jeweils gegebenenfalls substituiertes Phenylcarbonyl, Phenylalkylcarbonyl, Phenylalkenylcarbonyl, Furylcarbonyl, Thienylcarbonyl, Pyridylcarbonyl, Phenoxyalkylcarbonyl, Alkylcarbonyloxyalkylcarbonyl, Alkoxycarbonylcarbonyl oder Phenoxycarbonyl, für Alkylsulfonyl, Halogenalkylsulfonyl, oder gegebenenfalls substituiertes Phenylsulfonyl, für Alkylaminocarbonyl oder gegebenenfalls substituiertes Phenylaminocarbonyl steht,

A für Alkandiyl steht und

Z für gegebenenfalls substituiertes Aryl oder gegebenenfalls substituiertes Hetaryl steht.

2. Herbizide Mittel gemäß Anspruch 1, dadurch gekennzeichnet, daß in Formel (1A)

$R^1$, $R^2$,$R^3$ und $R^4$ unabhängig voneinander für Wasserstoff, Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, jeweils geradkettiges oder verzweigtes Alkoxy oder Alkylthio mit 1 bis 4 Kohlenstoffatomen oder jeweils geradkettiges oder verzweigtes Halogenalkoxy oder Halogenalkylthio mit 1 bis 4 Kohlenstoffatomen und 1 bis 9 gleichen oder verschiedenen Halogenatomen stehen,

$R^{5-2}$ für Cyano, geradkettiges oder verzweigtes Alkylcarbonyl mit 1 bis 20 Kohlenstoffatomen im Alkylteil, für Cycloalkylcarbonyl mit 3 bis 6 Kohlenstoffatomen im Cycloalkylteil, für jeweils geradkettiges oder verzweigtes Alkenylcarbonyl oder Alkinylcarbonyl mit jeweils 2 bis 20 Kohlenstoffatomen im Alkenyl- oder Alkinylteil, für geradkettiges oder verzweigtes Halogenalkylcarbonyl mit 1 bis 20 Kohlenstoffatomen im Alkylteil und 1 bis 41 gleichen oder verschiedenen Halogenatomen, für geradkettiges oder verzweigtes Alkoxyalkylcarbonyl mit jeweils 1 bis 4 Kohlenstoffatomen im Alkoxy- und im Alkylteil, für geradkettiges oder verzweigtes Halogenalkenylcarbonyl mit 2 bis 20 Kohlenstoffatomen im

Alkenylteil und 1 bis 39 gleichen oder verschiedenen Halogenatomen, für geradkettiges oder verzweigtes Halogenalkinylcarbonyl mit 2 bis 20 Kohlenstoffatomen im Alkinylteil und 1 bis 37 gleichen oder verschiedenen Halogenatomen, für geradkettiges oder verzweigtes Alkoxycarbonyl mit 1 bis 20 Kohlenstoffatomen im Alkoxyteil, für geradkettiges oder verzweigtes Alkylcarbonyloxyalkylcarbonyl mit jeweils 1 bis 4 Kohlenstoffatomen in den Alkylteilen, für Alkoxycarbonylcarbonyl mit 1 bis 4 Kohlenstoffatomen im Alkoxyteil, für geradkettiges oder verzweigtes Alkylthiocarbonyl mit 1 bis 20 Kohlenstoffatomen im Alkylteil für jeweils gegebenenfalls einfach oder mehrfach, gleich oder verschieden substituiertes Phenylcarbonyl, Phenylmethylcarbonyl, Phenylethylcarbonyl, Phenylpropylcarbonyl, Phenylethenylcarbonyl, Furylcarbonyl, Thienylcarbonyl, Pyridylcarbonyl, Phenoxymethylcarbonyl, Phenoxyethylcarbonyl, Phenoxycarbonyl oder Phenylaminocarbonyl steht, wobei als Substituenten jeweils infrage kommen: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen sowie geradkettiges oder verzweigtes Halogenalkoxy mit 1 bis 4 Kohlenstoffatomen und 1 bis 9 gleichen oder verschiedenen Halogenatomen;

$R^{5-2}$ weiterhin für geradkettiges oder verzweigtes Alkylsulfonyl mit 1 bis 4 Kohlenstoffatomen, für geradkettiges oder verzweigtes Halogenalkylsulfonyl mit 1 bis 4 Kohlenstoffatomen und 1 bis 9 gleichen oder verschiedenen Halogenatomen, für Alkylaminocarbonyl mit 1 bis 20 Kohlenstoffatomen im geradkettigen oder verzweigten Alkylteil oder für gegebenenfalls einfach oder mehrfach, gleich oder verschieden substituiertes Phenylsulfonyl steht, wobei als Phenylsubstituenten jeweils infrage kommen: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen sowie Alkoxycarbonyl mit 1 bis 4 Kohlenstoffatomen im geradkettigen oder verzweigten Alkoxyteil,

A für geradkettiges oder verzweigtes Alkandiyl mit 1 bis 4 Kohlenstoffatomen steht und

Z für jeweils gegebenenfalls einfach oder mehrfach, gleich oder verschieden substituiertes Phenyl, Pyridinyl, Thienyl, Thiazolyl, Isothiazolyl, Isoxazolyl oder für Oxa-2,4-diazolyl steht, wobei als Substituenten jeweils infrage kommen: Nitro, Cyano, Halogen, geradkettiges oder verzweigtes Alkyl mit 1 bis 4 Kohlenstoffatomen, jeweils geradkettiges oder verzweigtes Halogenalkyl oder Halogenalkylthio mit jeweils 1 bis 4 Kohlenstoffatomen und jeweils 1 bis 9 gleichen oder verschiedenen Halogenatomen, jeweils geradkettiges oder verzweigtes Alkoxy, Alkylthio oder Alkylsulfonyl mit jeweils 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Alkoxycarbonyl mit 1 bis 4 Kohlenstoffatomen im Alkoxyteil sowie gegebenenfalls einfach oder mehrfach, gleich oder verschieden durch Halogen, $C_1$-$C_4$-Alkyl oder Halogen-$C_1$-$C_4$-alkyl substituiertes Phenoxy.

3. Herbizide Mittel gemäß Anspruch 1, dadurch gekennzeichnet, daß in der Formel (IA)

$R^1$ für Wasserstoff steht,

$R^2$ für Wasserstoff, Fluor, Chlor, Brom, Methyl, Ethyl oder Trifluormethyl steht,

$R^3$ für Wasserstoff, Methyl, Ethyl oder Trifluormethyl steht,

$R^4$ für Wasserstoff steht,

$R^{5-2}$ für Cyano, geradkettiges oder verzweigtes Alkylcarbonyl mit 1 bis 10, vorzugsweise 1 bis 5, Kohlenstoffatomen im Alkylteil, für $C_3$-$C_6$-Cycloalkylcarbonyl, für jeweils geradkettiges oder verzweigtes Alkenylcarbonyl oder Alkinylcarbonyl mit jeweils 2 bis 10, vorzugsweise 2 bis 5, Kohlenstoffatomen im Alkenyl- oder Alkinylteil, für geradkettiges oder verzweigtes

Halogenalkylcarbonyl mit 1 bis 10, vorzugsweise 1 bis 4, Kohlenstoffatomen und 1 bis 21, vorzugsweise 1 bis 9 Fluor- und/oder Chloratomen, für Methoxyacetyl, für Acetyloxyacetyl, für geradkettiges oder verzweigtes Halogenalkenylcarbonyl mit 2 bis 10, vorzugsweise 2 bis 4, Kohlenstoffatomen im Alkenylteil und 1 bis 19, vorzugsweise 1 bis 3 Fluor- und/oder Chloratomen, geradkettiges oder verzweigtes Alkinylcarbonyl mit 2 bis 10, vorzugsweise 2 bis 4, Kohlenstoffatomen im Alkinylteil und 1 bis 17, vorzugsweise 1 bis 3 Fluor- und/oder Chloratomen, für geradkettiges oder verzweigtes Alkoxycarbonyl mit 1 bis 10, vorzugsweise 1 bis 5, Kohlenstoffatomen im Alkoxyteil, für geradkettiges oder verzweigtes Alkylthiocarbonyl mit 1 bis 10, vorzugsweise 1 bis 5, Kohlenstoffatomen im Alkylteil, für jeweils gegebenenfalls ein- oder zweifach, gleich oder verschieden durch Fluor, Chlor, Brom, Cyano, Nitro, Methyl, Trifluormethyl oder Methoxy substituiertes Phenylcarbonyl, Phenylmethylcarbonyl, Phenylethylcarbonyl, Phenylpropylcarbonyl, Phenylethenylcarbonyl, Furylcarbonyl, Thienylcarbonyl, Pyridylcarbonyl, Phenoxymethylcarbonyl, Phenoxyethylcarbonyl, Phenoxycarbonyl oder Phenylaminocarbonyl, für Methylsulfonyl, Ethylsulfonyl, Trifluormethylsulfonyl, für geradkettiges oder verzweigtes Alkylaminocarbonyl mit 1 bis 10, vorzugsweise 1 bis 5, Kohlenstoffatomen im Alkylteil, oder für gegebenenfalls ein- oder zweifach, gleich oder verschieden durch Fluor, Chlor, Cyano, Nitro, Methyl, Trifluormethyl, Methoxycarbonyl oder Ethoxycarbonyl substituiertes Phenylsulfonyl steht,

A für Methandiyl oder Ethandiyl, insbesondere Methandiyl, steht und

Z für jeweils gegebenenfalls einfach oder zweifach, gleich oder verschieden substituiertes Phenyl, Pyridinyl, Thienyl, Thiazolyl, Isothiazolyl, Isoxazolyl oder Oxa-2,4-diazolyl steht, wobei als Substituenten jeweils infrage kommen: Nitro, Cyano, Fluor, Chlor, Brom, Methyl, Ethyl, Trifluormethyl, Difluormethyl, Methylthio, Methylsulfonyl, Trifluormethylthio, Difluormethylthio, Methoxy, Ethoxy, Trifluormethoxy, Difluormethoxy, Methoxycarbonyl, Ethoxycarbonyl sowie Phenoxy.

4. Verfahren zur Bekämpfung von unerwünschten Pflanzen, dadurch gekennzeichnet, daß man 2-Iminopyridin-Derivate der Formel (IA) gemäß Anspruch 1 auf die Pflanzen und/oder ihren Lebensraum einwirken läßt.

5. Verwendung von 2-Iminopyridin-Derivaten der Formel (IA) gemäß Anspruch 1 zur Bekämpfung von unerwünschten Pflanzen.

6. Verfahren zur Herstellung von herbiziden Mitteln, dadurch gekennzeichnet, daß man 2-Iminopyridin-Derivate der Formel (IA) gemäß Anspruch 1 mit Streckmitteln und/oder oberflächenaktiven Substanzen vermischt.

7. 2-Iminopyridin-Derivate der allgemeinen Formel (I),

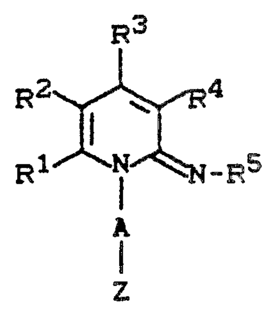

(I)

in welcher

$R^1$, $R^2$, $R^3$ und $R^4$ unabhängig voneinander für Wasserstoff, Halogen, Alkyl, Halogenalkyl, Alkoxy, Halogenalkoxy, Alkylthio oder Halogenalkylthio stehen,

$R^5$ für Alkylcarbonyl, Cycloalkylcarbonyl, Alkenylcarbonyl, Alkinylcarbonyl, Halogenalkylcarbonyl, Alkoxyalkylcarbonyl, Halogenalkenylcarbonyl, Halogenalkinylcarbonyl, Alkoxycarbonyl, Alkylcarbonyloxyalkylcarbonyl, Alkoxycarbonylcarbonyl, Alkylthiocarbonyl, für jeweils gegebenenfalls substituiertes Phenylcarbonyl, Phenylalkylcarbonyl, Phenylalkenylcarbonyl, Furylcarbonyl, Thienylcarbonyl, Pyridylcarbonyl, Phenoxyalkylcarbonyl oder Phenoxycarbonyl, für Alkylsulfonyl, Halogen alkylsulfonyl, gegebenenfalls substituiertes Phenylsulfonyl, für Alkylaminocarbonyl oder gegebenenfalls substituiertes Phenylaminocarbonyl steht,

A für Alkandiyl steht und

Z für gegebenenfalls substituiertes Aryl oder gegebenenfalls substituiertes Hetaryl steht.

**8.** 2-Iminopyridin-Derivate der Formel (I) gemäß Anspruch 7, bei welchen

$R^1$, $R^2$, $R^3$ und $R^4$ unabhängig voneinander für Wasserstoff, Halogen, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, jeweils geradkettiges oder verzweigtes Alkoxy oder Alkylthio mit 1 bis 4 Kohlenstoffatomen, jeweils geradkettiges oder verzweigtes Halogenalkoxy oder Halogenalkylthio mit 1 bis 4 Kohlenstoffatomen und 1 bis 9 gleichen oder verschiedenen Halogenatomen stehen,

$R^5$ für geradkettiges oder verzweigtes Alkylcarbonyl mit 1 bis 20 Kohlenstoffatomen im Alkylteil, für Cycloalkylcarbonyl mit 3 bis 6 Kohlenstoffatomen im Cycloalkylteil für jeweils geradkettiges oder verzweigtes Alkenyl carbonyl oder Alkinylcarbonyl mit jeweils 2 bis 20 Kohlenstoffatomen im Alkenyl- oder Alkinylteil, für geradkettiges oder verzweigtes Halogenalkylcarbonyl mit 1 bis 20 Kohlenstoffatomen im Alkylteil und 1 bis 41 gleichen oder verschiedenen Halogenatomen, für geradkettiges oder verzweigtes Alkoxyalkylcarbonyl mit jeweils 1 bis 4 Kohlenstoffatomen im Alkoxy- und im Alkylteil, für geradkettiges oder verzweigtes Halogenalkenylcarbonyl mit 2 bis 20 Kohlenstoffatomen im Alkenylteil und 1 bis 39 gleichen oder verschiedenen Halogenatomen, für geradkettiges oder verzweigtes Halogenalkinylcarbonyl mit 2 bis 20 Kohlenstoffatomen im Alkinylteil und 1 bis 37 gleichen oder verschiedenen Halogenatomen, für geradkettiges oder verzweigtes Alkoxycarbonyl mit 1 bis 20 Kohlenstoffatomen im Alkoxyteil, für geradkettiges oder verzweigtes Alkylcarbonyloxyalkylcarbonyl mit jeweils 1 bis 4 Kohlenstoffatomen in den Alkylteilen, für Alkoxycarbonylcarbonyl mit 1 bis 4 Kohlenstoffatomen im Alkoxyteil, für geradkettiges oder verzweigtes Alkylthiocarbonyl mit 1 bis 20 Kohlenstoffatomen im Alkylthioteil, für jeweils gegebenenfalls einfach oder mehrfach, gleich oder verschieden substituiertes Phenylcarbonyl, Phenylmethylcarbonyl, Phenylethylcarbonyl, Phenylpropylcarbonyl, Phenylethenylcarbonyl, Furylcarbonyl, Thienylcarbonyl, Pyridylcarbonyl, Phenoxymethylcarbonyl, Phenoxy-ethylcarbonyl, Phenoxycarbonyl, oder Phenylaminocarbonyl steht, wobei als Substituenten jeweils infrage kommen: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen sowie geradkettiges oder verzweigtes Halogenalkoxy mit 1 bis 4 Kohlenstoffatomen und 1 bis 9 gleichen oder verschiedenen Halogenatomen; $R^5$ weiterhin für geradkettiges oder verzweigtes Alkylsulfonyl mit 1 bis 4 Kohlenstoffatomen, für geradkettiges oder verzweigtes Halogenalkylsulfonyl mit 1 bis 4 Kohlenstoffatomen und 1 bis 9 gleichen oder verschiedenen Halogenatomen, für Alkylaminocarbonyl mit 1 bis 20 Kohlenstoffatomen im geradkettigen oder verzweigten Alkylteil oder für gegebenenfalls einfach oder mehrfach, gleich oder verschieden substituiertes Phenylsulfonyl steht, wobei als Phenylsubstituenten jeweils infrage kommen: Halogen, Cyano, Nitro, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 gleichen oder verschiedenen Halogenatomen, geradkettiges oder verzweigtes Alkoxy mit 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen sowie Alkoxycarbonyl mit 1 bis 4 Kohlenstoffatomen im

geradkettigen oder verzweigten Alkoxyteil,

A für geradkettiges oder verzweigtes Alkandiyl mit 1 bis 4 Kohlenstoffatomen steht und

Z für jeweils gegebenenfalls einfach oder mehrfach, gleich oder verschieden substituiertes Phenyl, Pyridinyl, Thienyl, Thiazolyl, Isothiazolyl, Isoxazolyl oder für Oxa-2,4-diazolyl steht, wobei als Substituenten jeweils infrage kommen: Nitro, Cyano, Halogen, geradkettiges oder verzweigtes Alkyl mit 1 bis 4 Kohlenstoffatomen, jeweils geradkettiges oder verzweigtes Halogenalkyl oder Halogenalkylthio mit jeweils 1 bis 4 Kohlenstoffatomen und jeweils 1 bis 9 gleichen oder verschiedenen Halogenatomen, jeweils geradkettiges oder verzweigtes Alkoxy, Alkylthio oder Alkylsulfonyl mit jeweils 1 bis 4 Kohlenstoffatomen, geradkettiges oder verzweigtes Halogenalkoxy mit 1 bis 4 Kohlenstoffatomen und 1 bis 9 gleichen oder verschiedenen Halogenatomen, geradkettiges oder verzweigtes Alkoxycarbonyl mit 1 bis 4 Kohlenstoffatomen im Alkoxyteil sowie gegebenenfalls einfach oder mehrfach, gleich oder verschieden durch Halogen, $C_1$-$C_4$-Alkyl und Halogen-$C_1$-$C_4$-alkyl substituiertes Phenoxy.

**9.** Verfahren zur Herstellung von 2-Iminopyridin-Derivaten der allgemeinen Formel (I)

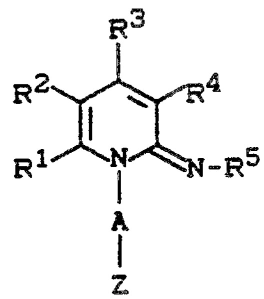

(I)

in welcher
$R^1$, $R^2$, $R^3$, $R^4$, $R^5$, A und Z die in Anspruch 7 angegebene Bedeutung haben, dadurch gekennzeichnet, daß man 1,2-Dihydropyridiniminiumsalze der allgemeinen Formel (II),

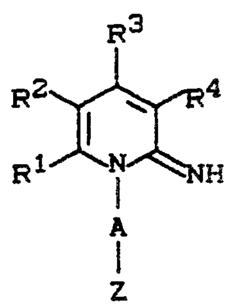

x HX (II)

in welcher
$R^1$, $R^2$, $R^3$, $R^4$, A und Z die in Anspruch 7 angegebene Bedeutung haben und

HX für das Äquivalent einer anorganischen oder organischen Säure steht,

(a) mit Halogenverbindungen der allgemeinen Formel (III),

$$R^{5-1}-X^1 \quad (III)$$

in welcher

$R^{5-1}$ für Alkylcarbonyl, Cycloalkylcarbonyl, Alkenylcarbonyl, Alkinylcarbonyl, Halogenalkylcarbonyl, Alkoxyalkylcarbonyl, Halogenalkenylcarbonyl, Halogenalkinylcarbonyl, Alkoxycarbonyl, Alkylthiocarbonyl, für jeweils gegebenenfalls substituiertes Phenylcarbonyl, Phenylalkylcarbonyl, Phenylalkenylcarbonyl, Furylcarbonyl, Thienylcarbonyl, Pyridylcarbonyl, Phenoxyalkylcarbonyl, Alkylcarbonyloxyalkylcarbonyl, Alkoxycarbonylcarbonyl oder Phenoxycarbonyl, für Alkylsulfonyl, Halogenalkylsulfonyl, oder gegebenenfalls substituiertes Phenylsulfonyl, steht,

$X^1$ für Halogen steht,

und für den Fall, daß $R^{5-1}$ für Alkylcarbonyl, Alkenylcarbonyl, Alkinylcarbonyl, Halogenalkylcarbonyl, Halogenalkenylcarbonyl, Halogenalkinylcarbonyl oder für gegebenenfalls substituiertes Phenyl carbonyl steht, mit den entsprechenden Carbonsäureanhydriden,

gegebenenfalls in Gegenwart einer Base und gegebenenfalls in Gegenwart eines Verdünnungsmittels umsetzt oder

(b) mit Isocyanaten der allgemeinen Formel (IV),

$$R^6\text{-NCO} \quad (IV)$$

in welcher

$R^6$ für Alkyl oder für gegebenenfalls substituiertes Phenyl steht,

gegebenenfalls in Gegenwart einer Base und gegebenenfalls in Gegenwart eines Verdünnungsmittels umsetzt.